(19) Europäisches Patentamt / European Patent Office / Office européen des brevets

(11) **EP 3 276 571 A1**

(12) **EUROPEAN PATENT APPLICATION**
published in accordance with Art. 153(4) EPC

(43) Date of publication:
**31.01.2018 Bulletin 2018/05**

(51) Int Cl.:
**G06Q 50/22** (2018.01)

(21) Application number: **16768379.6**

(22) Date of filing: **07.03.2016**

(86) International application number:
**PCT/JP2016/056949**

(87) International publication number:
**WO 2016/152476 (29.09.2016 Gazette 2016/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **25.03.2015 JP 2015063084**

(71) Applicant: **FUJIFILM Corporation
Tokyo 106-8620 (JP)**

(72) Inventor: **KANADA, Shoji
Ashigarakami-gun
Kanagawa 258-8538 (JP)**

(74) Representative: **Klunker IP
Patentanwälte PartG mbB
Destouchesstraße 68
80796 München (DE)**

(54) **MEDICAL DIAGNOSIS ASSISTANCE DEVICE, METHOD FOR OPERATING MEDICAL DIAGNOSIS ASSISTANCE DEVICE, AND MEDICAL DIAGNOSIS ASSISTANCE SYSTEM**

(57) There are provided a medical examination assistance apparatus capable of easily determining the future medical condition of a medical examination target patient, an operation method of a medical examination assistance apparatus, and a medical examination assistance system. An acquisition unit 47 acquires comparative cases, which are medical examination data having the same clinical path as the medical examination target patient, from an electronic medical record DB 14. A classification unit 48 classifies the comparative cases into a good group and a poor group according to the length of a treatment period. A calculation unit 49 calculates a representative value representing each group from the examination values of the comparative cases belonging to each group. A determination unit 50 determines whether or not there is a significant difference between the groups by t test. A screen display control unit 51 generates a graph comparison display screen 21, on which line graphs 92 and 93 showing a time-series change in the representative value and a line graph 94 showing a time-series change in the examination value of the medical examination target patient overlap each other, in a case where the determination unit 50 determines that there is a significant difference.

FIG. 15

**Description**

**BACKGROUND OF THE INVENTION**

1. Field of the Invention

The present invention relates to a medical examination assistance apparatus, an operation method of a medical examination assistance apparatus, and a medical examination assistance system.

2. Description of the Related Art

In medical facilities, introduction of electronic medical records for digitizing and managing various kinds of medical examination data acquired in the course of medical examination for patients is under progress. The medical examination data includes: examination values (measurement values) of vital signs of patients; examination values of various medical examinations including subject examinations, such as biochemical examinations and blood tests, or physiological examinations, such as electroencephalographic examinations; and medical examination and treatment records in which types and doses of therapeutic drugs, the contents of medical examinations, the contents of treatment, names of diseases, order of various medical examinations, and events, such as admission and discharge that occurred in the course of medical examination for patients, are recorded. These various kinds of medical examination data are registered in the electronic medical record in time series together with the dates of acquisition dates, such as measurement dates, examination dates, administration dates, and medical examination dates.

For the examination values of vital signs, there is a plurality of items, such as patient's blood pressure, body temperature, heartbeat, pulse, and oxygen saturation. Also for the examination values of, for example, blood tests among the medical examinations, there is a plurality of items, such as a white blood cell count (WBC), a red blood cell count (RBC), hematocrit (Ht), and albumin (ALB). The examination values are registered in the electronic medical record so as to be distinguished for each of the plurality of items.

In a medical facility that has introduced the electronic medical record, it is possible to provide various kinds of information for assisting diagnosis to a doctor in the form of electronic data. For example, JP2014-109836A discloses a medical examination assistance apparatus that provides a graph, which shows a time-series change in the examination value of a medical examination target patient, so that the degree of progress of the disease of the patient to be examination is grasped or the treatment effect after the surgery for the medical examination target patient or after the administration of therapeutic drug is determined.

In JP2014-109836A, in order to make it possible to compare and study the medical condition of the medical examination target patient and the medical condition of another patient, a graph showing a time-series change in the examination value of a comparative case, which is the medical examination data of a patient having the same disease name as the medical examination target patient, is displayed in parallel with the graph of the examination value of the medical examination target patient, or these graphs are displayed so as to overlap each other. As the examination value of the comparative case, for example, a representative value such as an average value or a median of a plurality of examination values obtained in a plurality of vital sign measurements performed in the past or in a plurality of medical examinations is used.

**SUMMARY OF THE INVENTION**

For the doctor, it is very important to determine the future medical condition of the medical examination target patient with reference to the examination value. In particular, if it is predicted that the medical condition will become worse at the early stage of treatment, it is possible to perform appropriate treatment for the medical examination target patient earlier. Then, since the treatment outcome is improved eventually, the determination of the future medical condition is also very important for the medical examination target patient.

In JP2014-109836A, the graph of the examination value of the comparative case is displayed in parallel with the graph of the examination value of the medical examination target patient, or these graphs are displayed so as to overlap each other. However, since it is not known whether the treatment outcome of the comparative case is good or bad, it is difficult to determine the future medical condition only with the display. Therefore, in the current situation, the future medical condition of the medical examination target patient is determined empirically by the doctor.

Thus, in the current situation where the determination of the future medical condition depends on the experience of a doctor, an inexperienced doctor overlooks the sign of medical condition deterioration. For this reason, there is a risk of adverse effects such as prolonged treatment. Therefore, a mechanism capable of easily determining the future medical condition of the medical examination target patient without being influenced by the doctor's experience or and oversight has been requested.

It is an object of the present invention to provide a medical examination assistance apparatus capable of easily determining the future medical condition of a medical examination target patient, an operation method of a medical examination assistance apparatus, and a medical examination assistance system.

In order to achieve the aforementioned object, a medical examination assistance apparatus of the present invention comprises an acquisition unit, a classification unit, a calculation unit, a determination unit, and a screen display control unit. The acquisition unit acquires comparative cases, which are medical examination data of a target to be compared with a medical examination target patient, from a case database in which medical examination data including examination values registered in time series is registered for each patient. The classification unit classifies the comparative cases into a plurality of groups according to a treatment outcome. The calculation unit calculates a representative value representing a group based on the examination values of the comparative case belonging to the same group. The determination unit determines whether or not there is a significant difference between the plurality of groups. The screen display control unit performs control to display the representative value and the examination values of the medical examination target patient on a display screen so as to be comparable with each other in a case where the determination unit determines that there is a significant difference.

It is preferable that the screen display control unit displays a graph showing a time-series change in the representative value and a graph showing a time-series change in the examination value of the medical examination target patient or a graph showing a time-series change in an amount of change of the representative value from a reference value and a graph showing a time-series change in an amount of change of the examination value of the medical examination target patient from a reference value, on the display screen, so as to be comparable with each other. In this case, it is preferable that a reference date for displaying the graphs so as to overlap each other along a time axis is set. It is preferable that the screen display control unit displays the graphs of a period before and after the reference date so as to overlap each other.

It is preferable that the examination values have a plurality of items, the calculation unit calculates the representative value for each of the plurality of items, and the determination unit performs determination for each of the plurality of items.

It is preferable that the medical examination data includes a clinical path that summarizes a treatment plan for each patient and the comparative cases are the medical examination data having the same clinical path as the medical examination target patient. In the case of displaying the graphs so as to overlap each other along a time axis, it is preferable that an application start date of the clinical path is set as a reference date.

It is preferable that the medical examination data includes contents of surgery performed on a patient and the comparative cases are the medical examination data having the same surgical contents as the medical examination target patient. In the case of displaying the graphs so as to overlap each other along a time axis, it is preferable that a date of the surgery is set as a reference date.

It is preferable that the medical examination data includes a disease name of a patient and a therapeutic drug administered to a patient and the comparative cases are the medical examination data having the same disease name and therapeutic drug as the medical examination target patient. In the case of displaying the graphs so as to overlap each other along a time axis, it is preferable that an administration start date of the therapeutic drug is set as a reference date.

It is preferable that the comparative cases are determined according to a degree of similarity with the examination value of the medical examination target patient.

It is preferable that the classification unit classifies the comparative cases according to a length of a treatment period. Alternatively, it is preferable that the medical examination data includes a clinical path that summarizes a treatment plan for each patient and a variance that does not conform to the treatment plan and the classification unit classifies the comparative cases according to the variance.

It is preferable that the determination unit performs determination based on a statistic of the examination values of the comparative cases of each of the plurality of groups. It is preferable that the statistic is the number of examination values of the comparative cases and an average value and a variance of the examination values of the comparative cases and the determination unit performs determination by t test for testing whether or not there is a significant difference in the average value of each of the plurality of groups.

The screen display control unit does not display the representative value while the examination value of the medical examination target patient is within a specified range defined by the examination values of the comparative cases, which are classified into the group with the best treatment outcome by the classification unit, and displays the representative value in a case where the examination value of the medical examination target patient is outside the specified range.

It is preferable that the screen display control unit displays a normal range of the examination value on the display screen.

An operation method of a medical examination assistance apparatus of the present invention comprises an acquisition step, a classification step, a calculation step, a determination step, and a screen display control step. In the acquisition step, comparative cases, which are medical examination data of a target to be compared with a medical examination target patient, are acquired from a case database in which medical examination data including examination values registered in time series is registered for each patient. In the classification step, the comparative cases are classified into a plurality of groups according to a treatment outcome. In the calculation step, a representative value representing a group is calculated based on the examination values of the comparative case belonging to the same group. In the determination step, it is determined whether or not there is a significant difference between the plurality of groups. In the screen display control step, control to display the representative value and the examination values of the medical examination target patient on a display screen so as to be comparable with each other is performed in a case where it is determined that there is a significant difference in the determination step.

A medical examination assistance system of the present invention comprises the medical examination assistance apparatus according to any one of claims 1 to 18 and a client terminal connected to the medical examination assistance apparatus through a network.

According to the present invention, comparative cases that are medical examination data of a target to be compared with the medical examination target patient are acquired, the acquired comparative cases are classified into a plurality of groups according to the treatment outcome, a representative value representing the group is calculated, it is determined whether or not there is a significant difference between the plurality of groups, and control to display the representative value and the examination values of the medical examination target patient so as to be comparable with each other is performed in a case where it is determined that there is a significant difference. Therefore, it is possible to provide a medical examination assistance apparatus capable of easily determining the future medical condition of a medical examination target patient, an operation method of a medical examination assistance apparatus, and a medical examination assistance system.

**BRIEF DESCRIPTION OF THE DRAWINGS**

Fig. 1 is a diagram showing a medical examination assistance system.
Fig. 2 is a diagram showing various kinds of information transmitted and received between a client terminal, a medical examination assistance server, and a medical record DB server.
Fig. 3 is a diagram showing the contents of an electronic medical record DB.
Fig. 4 is a block diagram showing a computer that forms a client terminal, a medical examination assistance server, and a medical record DB server.
Fig. 5 is a block diagram showing the function of a CPU of a client terminal.
Fig. 6 is a block diagram showing the function of a CPU of a medical examination assistance server.
Fig. 7 is a block diagram showing the function of a CPU of a medical record DB server.
Fig. 8 is a diagram illustrating the acquisition function of an acquisition unit.
Fig. 9 is a graph showing a time-series change in the examination value of a certain item.
Fig. 10 is a diagram illustrating the classification function of a classification unit.
Fig. 11 is a diagram illustrating the calculation function of a calculation unit.
Fig. 12 is a diagram showing the contents of statistical data.
Fig. 13 is a diagram illustrating the determination function of a determination unit.
Fig. 14 is a diagram showing a medical examination data display screen.
Fig. 15 is a diagram showing a graph comparison display screen.
Fig. 16 is a diagram showing a graph comparison display screen in a case where a second distribution request including a plurality of items is received.
Fig. 17 is a flowchart showing the processing procedures of the CPU of the client terminal, the CPU of the medical examination assistance server, and the CPU of the medical record DB server.
Fig. 18 is a diagram illustrating the acquisition function of an acquisition unit in a second embodiment.
Fig. 19 is a diagram showing a graph comparison display screen in the second embodiment.
Fig. 20 is a diagram showing a disease name and therapeutic drug table.
Fig. 21 is a diagram illustrating the acquisition function of an acquisition unit in a third embodiment.
Fig. 22 is a diagram showing a graph comparison display screen in the third embodiment.
Fig. 23 is a diagram illustrating the search function of a search unit in a fourth embodiment.
Fig. 24 is a diagram illustrating the classification function of a classification unit in a fifth embodiment.
Fig. 25 is a diagram illustrating the calculation function of a calculation unit and the screen display control function of a screen display control unit in a sixth embodiment.
Fig. 26 is a diagram illustrating the calculation function of a calculation unit in a seventh embodiment.
Fig. 27 is a diagram illustrating the calculation function of the calculation unit in the seventh embodiment.

Fig. 28 is a diagram showing a graph comparison display screen in the seventh embodiment.

Fig. 29 is a diagram illustrating the classification function of a classification unit in an eighth embodiment.

Fig. 30 is a diagram showing a graph comparison display screen in the eighth embodiment.

## DESCRIPTION OF THE PREFERRED EMBODIMENTS

[First embodiment]

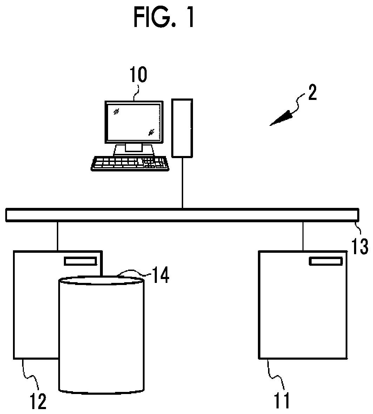

In Fig. 1, a medical examination assistance system 2 includes a client terminal 10 and a medical examination assistance server 11 corresponding to a medical examination assistance apparatus. The client terminal 10 and the medical examination assistance server 11 are connected to each other through a network 13, such as a local area network (LAN) provided in the medical facility. A medical record database (hereinafter, abbreviated as a database (DB)) server 12 is also connected to the network 13.

Each of the client terminal 10, the medical examination assistance server 11, and the medical record DB server 12 is configured by installing a control program, such as an operating system, or various application programs on a computer as a base, such as a personal computer, a server computer, or a workstation.

The medical examination assistance server 11 has a diagnostic assistance information providing function for providing diagnostic assistance information for assisting patient diagnosis. The medical record DB server 12 has a medical record management function for managing electronic medical records.

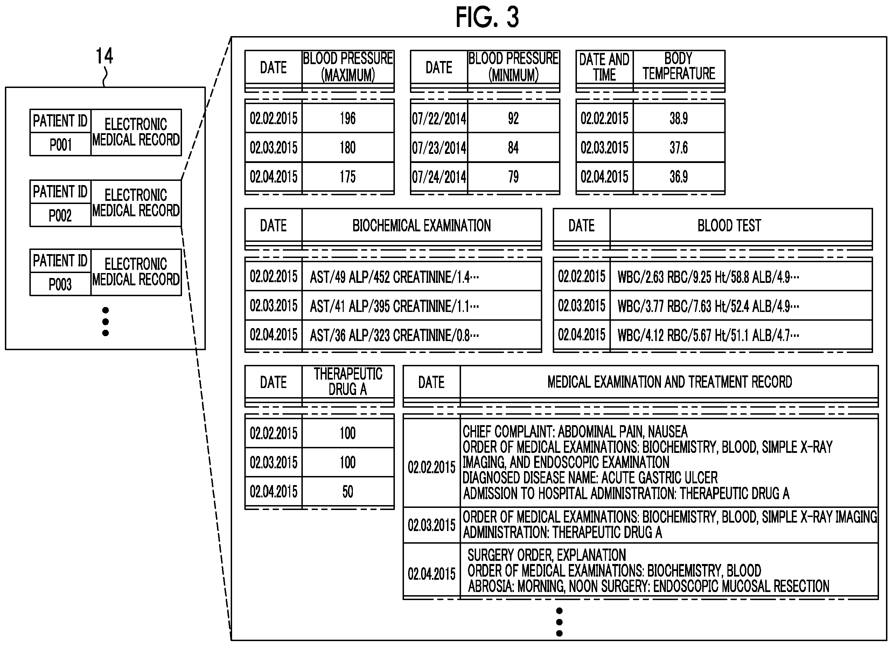

The client terminal 10 is operated by a medical staff of a medical facility, such as a doctor who performs medical examination for a patient or a laboratory technician who performs a medical examination. The client terminal 10 is used in the case of performing medical examination for a patient using various functions of the medical examination assistance server 11 and the medical record DB server 12. Specifically, the client terminal 10 is used in the case of viewing diagnostic assistance information or electronic medical records or in the case of inputting various kinds of medical examination data (refer to Fig. 3) in the electronic medical record. In Fig. 1, only one client terminal 10 is drawn. In practice, however, a plurality of client terminals 10 are provided for each medical department, such as internal medicine, surgery, examination department, and rehabilitation department, or each medical staff.

An electronic medical record DB 14 corresponding to a case database is provided in the medical record DB server 12. Electronic medical records are registered in the electronic medical record DB 14 so as to be searchable.

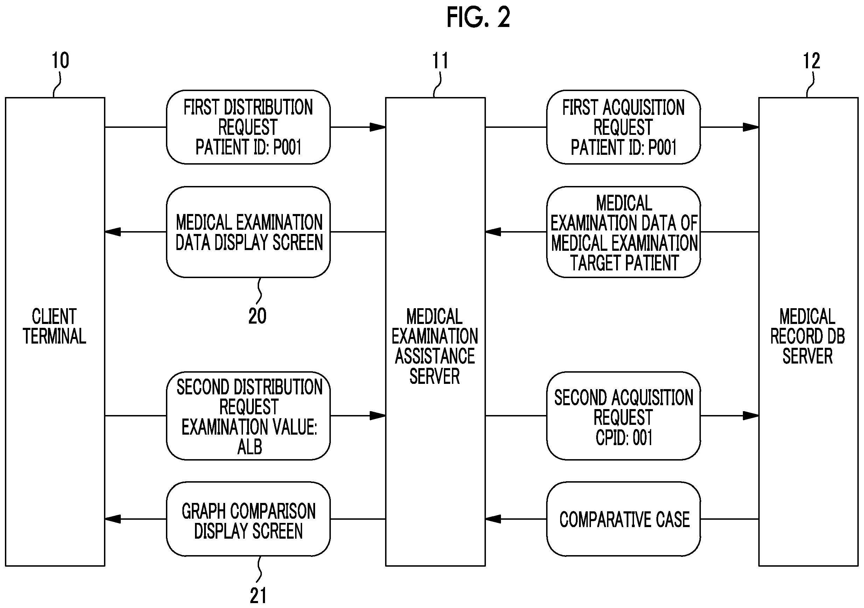

In Fig. 2, the client terminal 10 outputs a first distribution request and a second distribution request to the medical examination assistance server 11. The first distribution request includes patient identification data (ID) of the medical examination target patient who is an object of a medical examination. The patient ID is a symbol or a number for identifying an individual patient who visits a medical facility. In addition to the patient ID of the medical examination target patient, the second distribution request includes an item of examination value (only the item of examination value is shown in Fig. 2).

The medical examination assistance server 11 receives the first distribution request and the second distribution request from the client terminal 10. The medical examination assistance server 11 outputs a first acquisition request having the same contents as the first distribution request to the medical record DB server 12.

The medical examination assistance server 11 outputs the second acquisition request to the medical record DB server 12, for example, twice. The first-time second acquisition request includes a patient ID of the medical examination target patient. The second-time second acquisition request includes an ID (CPID) of a clinical path (hereinafter, abbreviated as a CP) adopted for the medical examination target patient (only the second-time second acquisition request is shown in Fig. 2).

The medical record DB server 12 receives the first acquisition request from the medical examination assistance server 11, and searches for medical examination data associated with the patient ID included in the first acquisition request (hereinafter, referred to as medical examination data of the medical examination target patient). The medical record DB server 12 outputs the medical examination data of the medical examination target patient to the medical examination assistance server 11.

In addition, the medical record DB server 12 receives the second acquisition request from the medical examination assistance server 11, searches for medical examination data of the medical examination target patient in response to the first-time second acquisition request, and searches for medical examination data (hereinafter, referred to as a comparative case) of a target to be compared with the medical examination target patient in response to the second-time second acquisition request. The medical record DB server 12 outputs the medical examination data of the medical examination target patient and the comparative case to the medical examination assistance server 11 (only the comparative case is shown in Fig. 2).

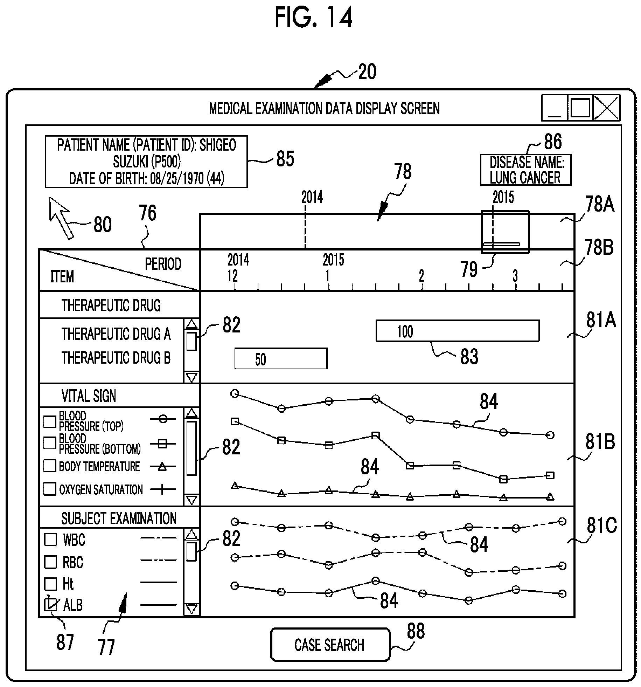

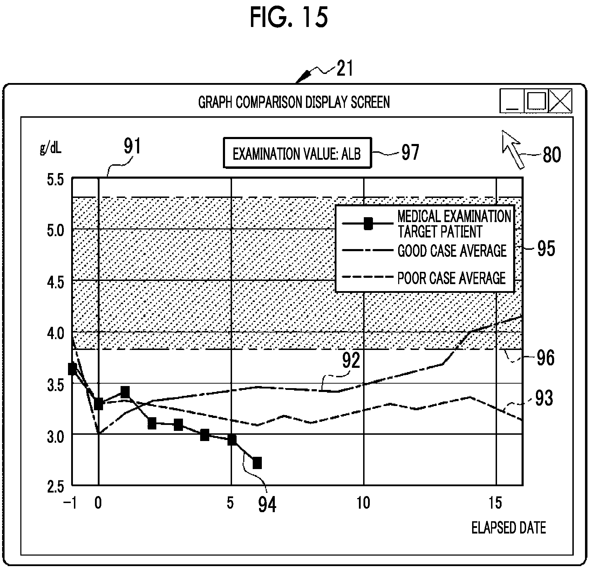

The medical examination assistance server 11 acquires the medical examination data of the medical examination target patient and the comparative case from the medical record DB server 12. The medical examination assistance server 11 generates a medical examination data display screen 20 (also refer to Fig. 14) based on the medical examination data of the medical examination target patient. In addition, the medical examination assistance server 11 generates a graph comparison display screen 21 (also refer to Fig. 15 or the like) as diagnostic assistance information based on the medical examination data of the medical examination target patient and the comparative case. The medical examination assistance server 11 outputs the generated display screens 20 and 21 to the client terminal 10 that is the output source of each distribution request.

The medical examination assistance server 11 distributes the display screens 20 and 21, for example, in the form of XML data for web distribution that is created by a markup language, such as Extensible Markup Language (XML). The client terminal 10 reproduces and displays the display screens 20 and 21 on the web browser based on the XML data. Instead of the XML, other data description languages, such as JavaScript (registered trademark) Object Notation (JSON), may be used.

Although not shown, in addition to the first distribution request and the second distribution request, the client terminal 10 outputs an electronic medical record registration request and an electronic medical record distribution request to the medical record DB server 12. The medical record DB server 12 registers the electronic medical record received by the registration request in a retrievable form in the electronic medical record DB 14, and manages the electronic medical record. In addition, the medical record DB server 12 searches for the electronic medical record designated by the distribution request from the electronic medical record DB 14, and outputs the searched electronic medical record to the client terminal 10 that is the output source of the distribution request.

In Fig. 3, in the electronic medical record DB 14, an electronic medical record is registered for each patient so as to be associated with a patient ID. The electronic medical record registered in the electronic medical record DB 14 can be searched based on the patient ID.

The electronic medical record includes various kinds of medical examination data. The medical examination data includes: examination values (measurement values) of vital signs, such as patient's blood pressure, body temperature, heartbeat, pulse, and oxygen saturation; examination values of various medical examinations including subject examinations, such as biochemical examinations and blood tests, or physiological examinations, such as electroencephalographic examinations; and medical examination and treatment records in which types and doses of therapeutic drugs, the contents of medical examinations, the contents of treatment, names of diseases, order of various medical examinations, and events, such as admission and discharge that occurred in the course of medical examination for patients, are recorded. These various kinds of medical examination data are registered in time series together with the dates of acquisition dates, such as measurement dates, examination dates, administration dates, and medical examination dates.

In Fig. 3, examination values of respective items of blood pressure (maximum), blood pressure (minimum), and body temperature are exemplified as examination values of vital signs. In addition, examination values of respective items, such as aspartate aminotransferase (AST), alkaline phosphatase (ALP), and creatinine of biochemical examinations, and examination values of respective items, such as WBC, RBC, Ht, and ALB of blood test, are exemplified as examination values of medical examinations, and the dose of therapeutic drug A is exemplified as the type and dose of therapeutic drug. In addition, a chief complaint such as abdominal pain and nausea obtained by interview, acute gastric ulcer of diagnosed disease name, order of medical examinations such as biochemistry, blood, simple X-ray imaging, and endoscopic examination, CP001 that is the CPID of the adopted CP, and the like are recorded as examples of the medical examination and treatment record.

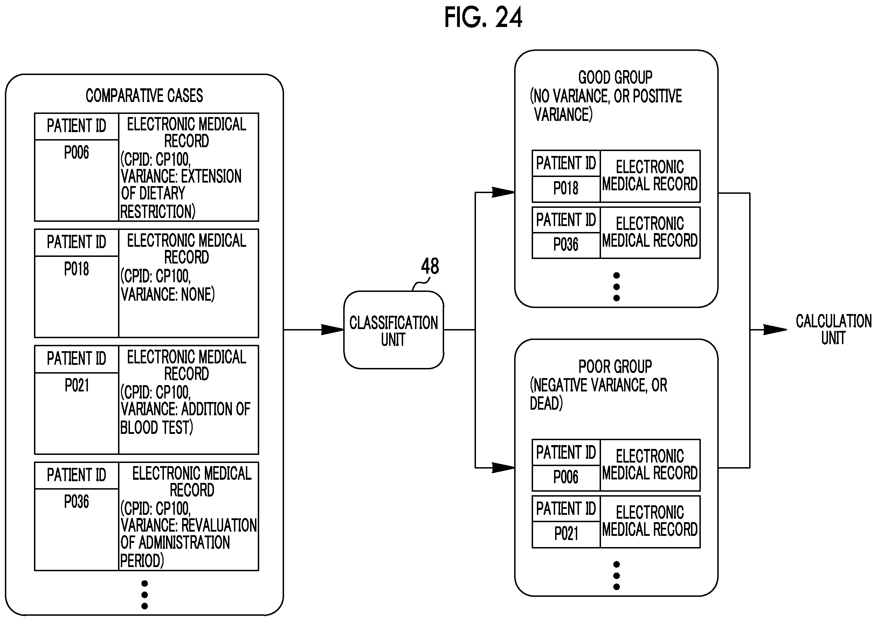

The CP is generated in advance for each disease type or each disease name in order to bring about an ideal treatment result for the medical examination target patient. The CP is a summary of a daily plan (treatment plan) of various treatments or the like to be performed for the medical examination target patient, such as medical examinations, surgery, rehabilitation, and meals. The medical staff performs treatment or the like along the treatment plan indicated by the CP. In a case where variance, which is treatment or the like that does not conform to the treatment plan indicated by the CP, occurs, the contents of the variance is recorded in the medical examination and treatment record by the medical staff (refer to Fig. 24).

The CPID is a symbol or a number for identifying each CP. The same CPID is assigned to CPs whose treatment plans generated for each disease type or disease name are the same.

As the medical examination data, in addition to the above examples, health management information measured on a daily basis by a patient using simple test equipment such as a blood pressure measuring instrument or a weighing scale in a home or genetic test information as a result of patient's genetic test may be included.

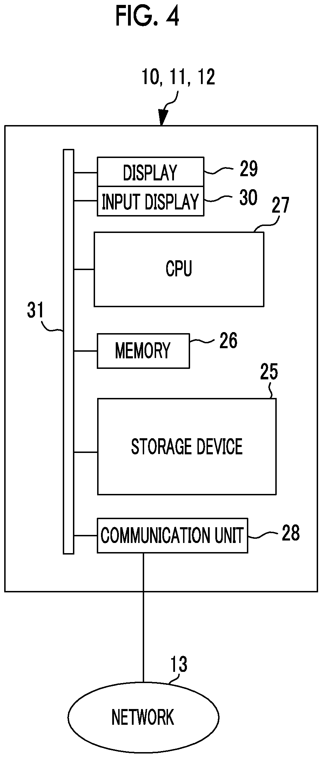

In Fig. 4, the basic configurations of computers that form the client terminal 10, the medical examination assistance server 11, and the medical record DB server 12 are the same, and each computer includes a storage device 25, a memory 26, a central processing unit (CPU) 27, a communication unit 28, a display 29, and an input device 30. These are connected to each other through a data bus 31.

The storage device 25 is a hard disk drive, which is built into a computer that forms the client terminal 10 or the like or which is connected to the computer through a cable or a network, or a disk array formed by connecting a plurality of hard disk drives. Control programs such as an operating system, various application programs, and display data of various operation screens associated with these programs are stored in the storage device 25.

The memory 26 is a work memory required in a case where the CPU 27 executes processing. The CPU 27 performs overall control of each unit of the computer by loading a program stored in the storage device 25 to the memory 26 and executing the processing according to the program.

The communication unit 28 is a network interface to perform transmission control of various kinds of information through the network 13. The display 29 displays various operation screens corresponding to the operation of the input device 30, such as a mouse or a keyboard. The operation screen has an operation function based on the graphical user interface (GUI). Each computer that forms the client terminal 10 or the like receives an input of an operation instruction from the input device 30 through the operation screen.

In the following explanation, for the sake of distinction, a suffix "A" is attached to the reference numeral of each unit of the computer that forms the client terminal 10, a suffix "B" is attached to the reference numeral of each unit of the computer that forms the medical examination assistance server 11, and a suffix "C" is attached to the reference numeral of each unit of the computer that forms the medical record DB server 12.

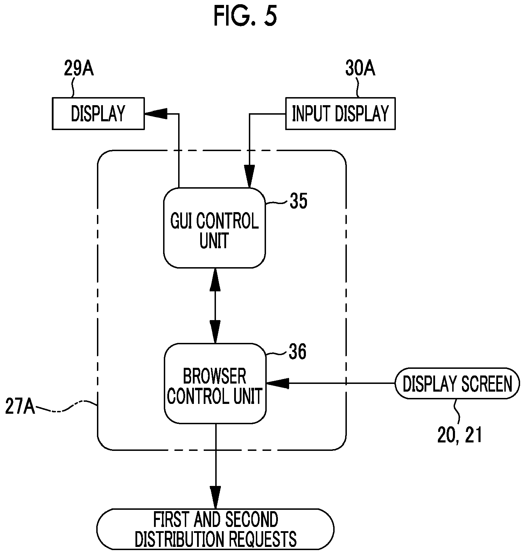

In Fig. 5, in a case where a web browser is started, the CPU 27A of the client terminal 10 cooperates with the memory 26 to function as a GUI control unit 35 and a browser control unit 36.

The GUI control unit 35 displays various operation screens on the display 29A, and receives an operation instruction that is input from the input device 30A through various operation screens. Operation instructions include an instruction to distribute the medical examination data display screen 20, specifically, an instruction to input the patient ID of the medical examination target patient, and an instruction to distribute the graph comparison display screen 21, specifically, an instruction to input an item of the examination value. The GUI control unit 35 outputs the received patient ID of the medical examination target patient and the received item of the examination value to the browser control unit 36.

The browser control unit 36 controls the operation of the web browser. The browser control unit 36 issues a first distribution request, which includes the patient ID of the medical examination target patient from the GUI control unit 35, and a second distribution request, which includes the item of the examination value from the GUI control unit 35, to the medical examination assistance server 11.

The browser control unit 36 receives XML data of each of the display screens 20 and 21 from the medical examination assistance server 11. The browser control unit 36 reproduces the display screens 20 and 21 to be displayed on the web browser based on the XML data, and outputs the display screens 20 and 21 to the GUI control unit 35. The GUI control unit 35 displays the display screens 20 and 21 on the display 29A.

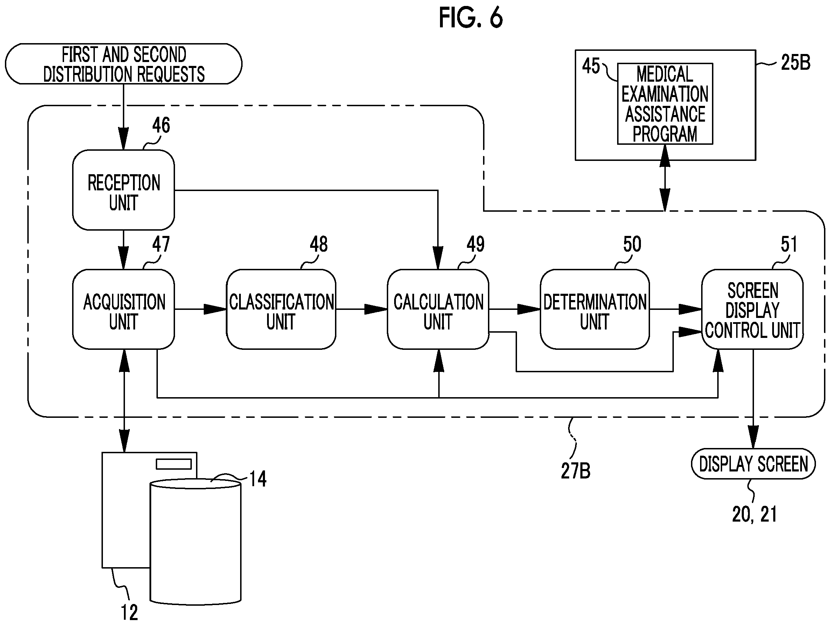

In Fig. 6, a medical examination assistance program 45 is stored in the storage device 25B of the medical examination assistance server 11. The medical examination assistance program 45 is an application program for operating a computer, which forms the medical examination assistance server 11, as a medical examination assistance apparatus, and corresponds to an operation program.

In a case where the medical examination assistance program 45 is started, the CPU 27B of the medical examination assistance server 11 cooperates with the memory 26 to function as a reception unit 46, an acquisition unit 47, a classification unit 48, a calculation unit 49, a determination unit 50, and a screen display control unit 51.

The reception unit 46 receives the first distribution request and the second distribution request from the client terminal 10. The reception unit 46 outputs the patient ID of the medical examination target patient, which is included in the first distribution request and the second distribution request, to the acquisition unit 47. In addition, the reception unit 46 outputs the item of the examination value included in the second distribution request to the calculation unit 49.

The acquisition unit 47 issues a first acquisition request, which includes the patient ID of the medical examination target patient, and a second acquisition request, which includes the patient ID of the medical examination target patient and the CPID of the medical examination target patient, to the medical record DB server 12.

The acquisition unit 47 acquires the medical examination data of the medical examination target patient and the comparative case that are output from the medical record DB server 12 in response to the first acquisition request and the second acquisition request. The acquisition unit 47 outputs the medical examination data of the medical examination target patient to the screen display control unit 51, and outputs the comparative case to the classification unit 48 and the calculation unit 49.

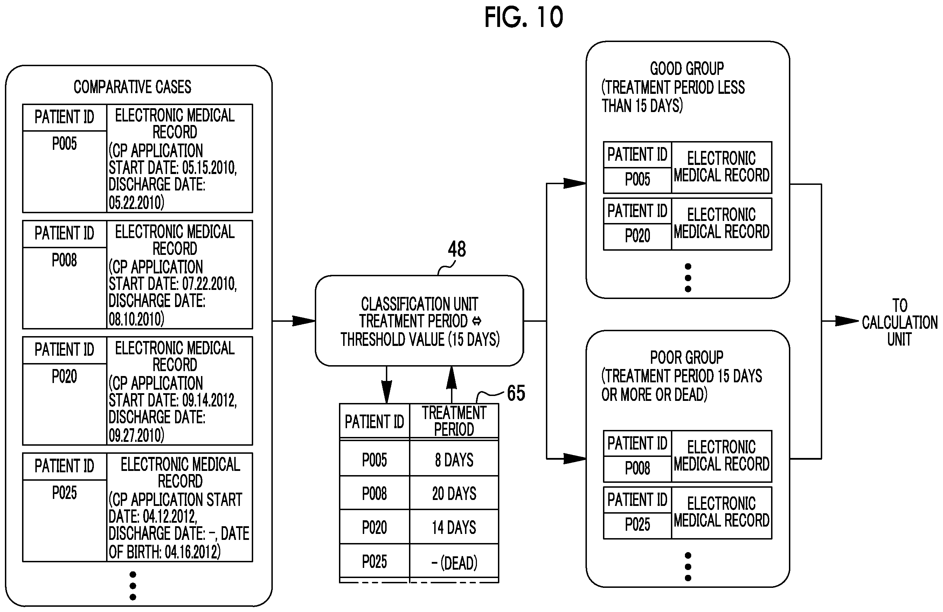

The classification unit 48 classifies the comparative case from the acquisition unit 47 into two groups of a good group and a poor group according to the treatment outcome (refer to Fig. 10). The classification unit 48 outputs the classification result to the calculation unit 49.

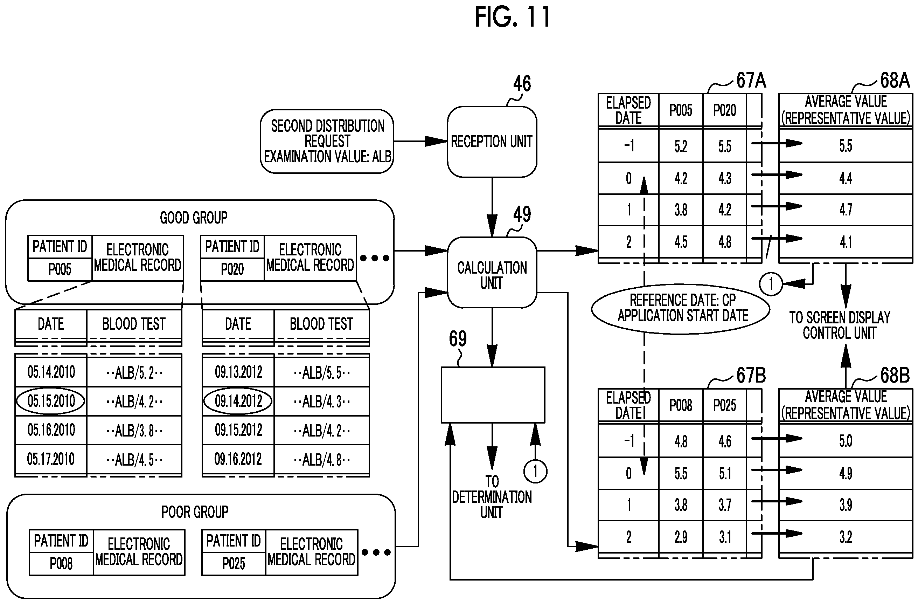

The calculation unit 49 calculates a representative value representing a group based on the examination values of the comparative case belonging to the same group (refer to Fig. 11). The calculation unit 49 calculates a representative value for the examination value of the item included in the second distribution request. The calculation unit 49 outputs the calculation result to the determination unit 50 and the screen display control unit 51.

The determination unit 50 determines whether or not there is a significant difference between the good group and the poor group for the examination value of the item included in the second distribution request. The determination unit 50 outputs the determination result to the screen display control unit 51.

The screen display control unit 51 generates the medical examination data display screen 20 based on the medical examination data of the medical examination target patient from the acquisition unit 47. In a case where the determination unit 50 determines that there is a significant difference, the screen display control unit 51 generates the graph comparison display screen 21 based on the medical examination data of the medical examination target patient from the acquisition unit 47 and the representative value from the calculation unit 49. The screen display control unit 51 outputs the XML data of the generated display screens 20 and 21 to the client terminal 10 that is the output source of each distribution request.

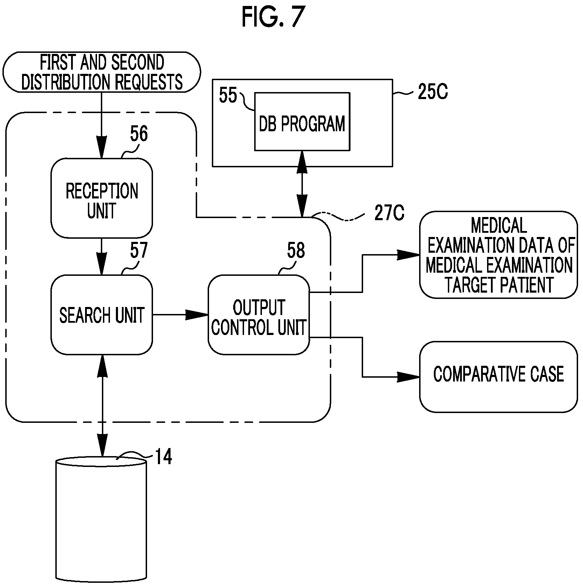

In Fig. 7, a DB program 55 is stored in the storage device 25C of the medical record DB server 12. In a case where the DB program 55 is started, the CPU 27C of the medical record DB server 12 cooperates with the memory 26 to function as a reception unit 56, a search unit 57, and an output control unit 58.

The reception unit 56 receives the first acquisition request and the second acquisition request from the medical examination assistance server 11. The reception unit 56 outputs to the search unit 57 the patient ID of the medical examination target patient included in the first acquisition request, the patient ID of the medical examination target patient included in the first-time second acquisition request, and the CPID of the medical examination target patient included in the second-time second acquisition request.

The search unit 57 searches for the medical examination data of the medical examination target patient from the electronic medical record DB 14 in response to the first acquisition request and the first-time second acquisition request, and searches for a comparative case in response to the second-time second acquisition request. The search unit 57 outputs the medical examination data of the medical examination target patient and the comparative case, which have been searched, to the output control unit 58. The output control unit 58 outputs the medical examination data of the medical examination target patient and the comparative case to the medical examination assistance server 11.

Although not shown, the reception unit 56 also receives a request for registration of the electronic medical record and a request for distribution of the electronic medical record. The search unit 57 searches for the electronic medical record from the electronic medical record DB 14 in response to the request for distribution of the electronic medical record. In addition, the search unit 57 also has a registration function for registering the electronic medical record in the electronic medical record DB 14 in response to the request for registration of the electronic medical record. The output control unit 58 outputs the electronic medical record, which has been searched by the search unit 57, to the client terminal 10 that is the output source of the distribution request.

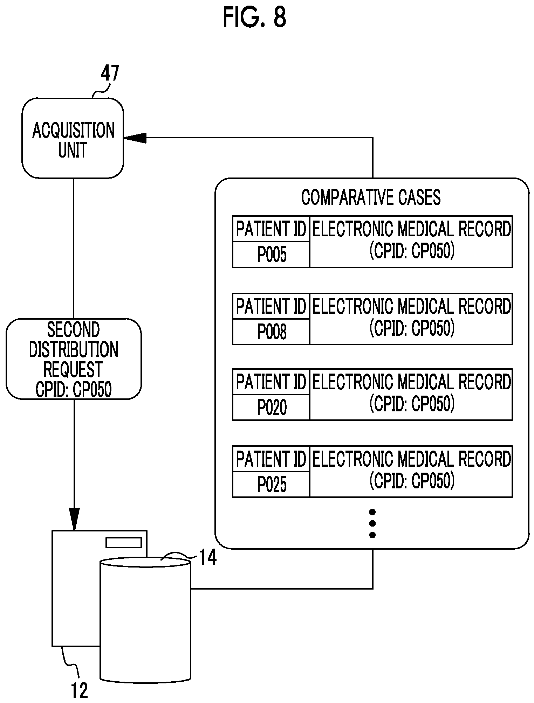

The acquisition unit 47 acquires medical examination data, which has the same clinical path (CP) as the medical examination target patient, as a comparative case. For example, in Fig. 8, in a case where the CPID "CP050" is recorded in the medical examination and treatment record of the electronic medical record of the medical examination target patient, the acquisition unit 47 outputs the second-time second acquisition request including the CPID "CP050" to the medical record DB server 12. In the medical record DB server 12, the search unit 57 searches for the medical examination data of the electronic medical record of patients (patients with patient IDs "P005", "P008", and the like) in which the CPID "CP050" is recorded in the medical examination and treatment record, as a comparative case, from the electronic medical record of each patient of the electronic medical record DB 14. As a result, the acquisition unit 47 acquires, medical examination data of the electronic medical record of a patient, in which the same CPID "CP050" as the medical examination target patient is recorded in the medical examination and treatment record, as a comparative case.

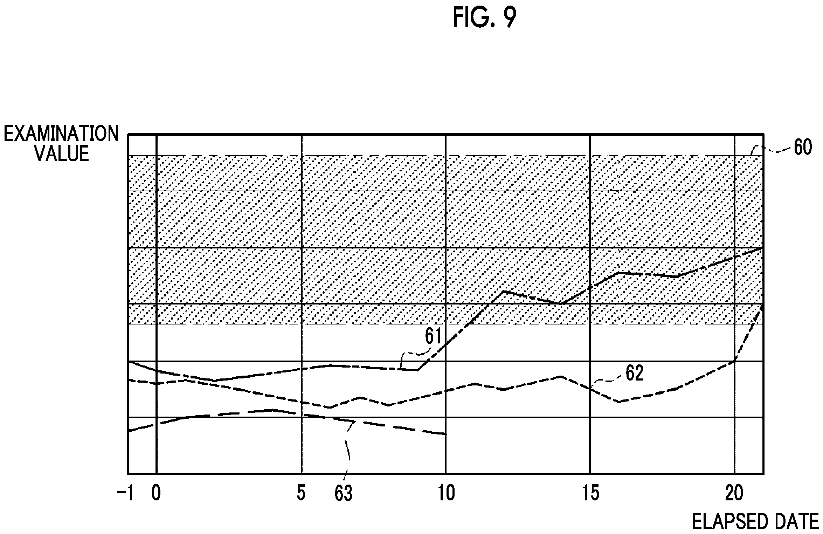

Fig. 9 is a graph showing a time-series change in the examination value of a certain item. The examination value is assigned to the vertical axis, and the elapsed date is assigned to the horizontal axis. The elapsed date "0" is the treatment start date of the medical examination target patient. Reference numeral 60 indicated by hatching and two-dot chain line shows the normal range of the examination value of the item.

In a line graph 61 indicated by the one-dot chain line, the examination value is within the normal range 60 on the elapsed date "11", and the examination value remains within the normal range 60 after the elapsed date "11". On the other hand, in a line graph 62 indicated by the narrow broken line, the examination value fall within the normal range 60 on the elapsed date "21". A line graph 63 indicated by the wide broken line is discontinued after the elapsed date "10" since the patient died on the elapsed date "10".

Here, it is assumed that a patient of a comparative case whose time-series change in the examination value is indicated by the line graph 61 was discharged on, for example, the elapsed date "12", and the patient of a comparative case whose time-series change in the examination value is indicated by the line graph 62 was discharged after, for example, the elapsed date "21". In this case, since the treatment period in the comparative case whose time-series change in the examination value is indicated by the line graph 61 is relatively shorter than that in the comparative case whose time-series change in the examination value is indicated by the line graph 62, it can be said that the treatment outcome in the comparative case whose time-series change in the examination value is indicated by the line graph 61 is better than that in the comparative case whose time-series change in the examination value is indicated by the line graph 62. Conversely, it can be said that the treatment outcome in the comparative case whose time-series change in the examination value is indicated by the line graph 62 is worse than that in the comparative case whose time-series change in the examination value is indicated by the line graph 61. In the comparative case whose time-series change in the examination value is indicated by the line graph 63, it can be said that the treatment outcome is bad since the patient is dead.

Thus, the treatment outcome is determined depending on the length of the treatment period and whether the patient is alive or dead. In the present embodiment, therefore, the classification unit 48 classifies comparative cases according to the length of the treatment period and whether the patient is alive or dead.

More specifically, as shown in Fig. 10, the classification unit 48 generates intermediate processing data 65 first. The intermediate processing data 65 is obtained by calculating the treatment period with the application start date of the CP recorded in the medical examination and treatment record as the start date of the treatment period and the discharge date as the end date of the treatment period and associating the calculated treatment period with the patient ID of the comparative case for each comparative case from the acquisition unit 47.

For example, in the case of a comparative case of patient ID "P005", since the application start date of the CP recorded in the medical examination and treatment records is "2010. 05. 15" and the discharge date is "2010. 05. 22", "eight days" is calculated as the treatment period. In a case where the patient is dead as in the comparative case of patient ID "P025", the classification unit 48 does not calculate the treatment period. The treatment period may be calculated with the discharge date as the end date of the treatment period as described above, or the treatment period may be calculated with the day before the discharge date as the end date of the treatment period. Instead of the discharge date, the administration end date of therapeutic drug may be used as the end date of the treatment period.

The classification unit 48 compares the calculated treatment period with a predetermined threshold value of the treatment period. The classification unit 48 classifies the comparative case into a good group in a case where the calculated treatment period is less than the threshold value, and classifies the comparative case into a poor group in a case where the calculated treatment period is equal to or greater than the threshold value or the patient is dead. Two threshold values to be compared with the calculated treatment period may be set as a threshold value for classification into a good group and a threshold value for classification into a poor group. In this case, the classification unit 48 classifies the comparative case into either a good group or a poor group by comparing the calculated treatment period with the two threshold values.

Fig. 10 exemplifies a case where "15 days" is set as the threshold value of the treatment period. In this case, since the treatment periods of the comparative cases of patient IDs "P005" and "P020" are "8 days" and "14 days", respectively, the classification unit 48 classifies the comparative cases of patient IDs "P005" and "P020" into a good group. On the other hand, since the treatment period of the comparative case of patient ID "P008" is "20 days" and the patient of patient ID "P025" is dead, the classification unit 48 classifies the comparative cases of patient IDs "P008" and "P025" into a poor group.

As the threshold value of the treatment period, for example, a standard treatment period defined by the CP or an average treatment period for each disease type or disease name announced by a public institution is set. In these cases, the threshold value of the treatment period is different for each disease type or disease name. Needless to say, the threshold value of the treatment period may be set uniformly regardless of the disease type or disease name.

Fig. 11 shows how the calculation unit 49 calculates a representative value of ALB examination values of comparative cases belonging to each of the good group and the poor group in a case where the item included in the second distribution request is ALB. Comparative cases, such as patient IDs "P005" and "P020" exemplified in Fig. 10, belong to the good group. The ALB examination value of each comparative case changes every acquisition date to "5.2, 4.2, 3.8, 4.5 ..." in the case of patient ID "P005", and changes every acquisition date to "5.5, 4.3, 4.2, 4.8 ..." in the case of patient ID "P020". Among the dates of the acquisition date, each of "2010. 05.15" and "2012. 09.14" surrounded by the solid ellipse indicates the application start date of the CP.

The calculation unit 49 sets the application start date of the CP as the elapsed date "0", that is, the reference date. For this reason, the elapsed date "-1" is the day before the reference date, the elapsed date "1" is the day after the reference date, and the elapsed date "2" is two days after the reference date. The calculation unit 49 generates intermediate processing data 67A, which is a collection of ALB examination values for each elapsed date of each comparative case belonging to the good group, and intermediate processing data 67B, which is a collection of ALB examination values for each elapsed date of each comparative case belonging to the poor group. For example, the ALB examination value for each elapsed date of each comparative case belonging to the good group is "5.2, 5.5, ..." in the case of elapsed date "-1", and is "4.2, 4.3, ..." in the case of elapsed date "0".

As shown in calculation results 68A and 68B, the calculation unit 49 calculates the average value of ALB examination values for each elapsed date as a representative value. The calculation result 68A is obtained by calculation based on the intermediate processing data 67A of the good group, and the calculation result 68B is obtained by calculation based on the intermediate processing data 67B of the poor group. For example, the representative value of the elapsed date "0" of the good group is "4.4", and the representative value of the elapsed date "0" of the poor group is "4.9". In this manner, one representative value is calculated for each elapsed date of the good group and the poor group.

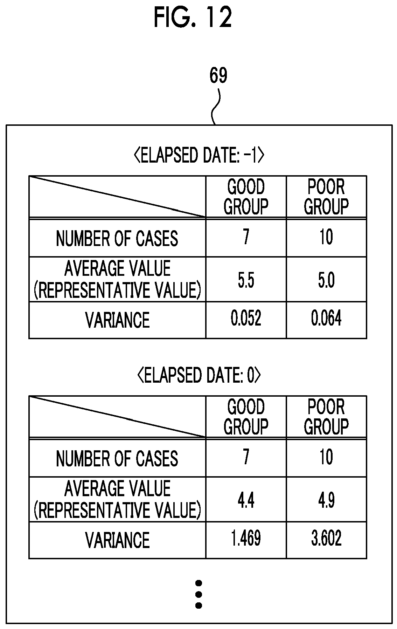

The calculation unit 49 outputs statistical data 69 including the average value, which is a representative value, to the determination unit 50. As shown in Fig. 12, the statistical data 69 is a collection of the number of examination values (here, ALB examination values) of the comparative cases of each of the good group and the poor group, the average value (representative value) of the examination values, and the variance of the examination values, for each elapsed date. For example, the number of ALB examination values of the elapsed date "-1" is "7" in the case of a good group and "10" in the case of a poor group, and the average value is "5.5" in the case of a good group and "5.0" in the case of a poor group as in Fig. 11. The variance is "0.052" in the case of a good group and "0.064" in the case of a poor group.

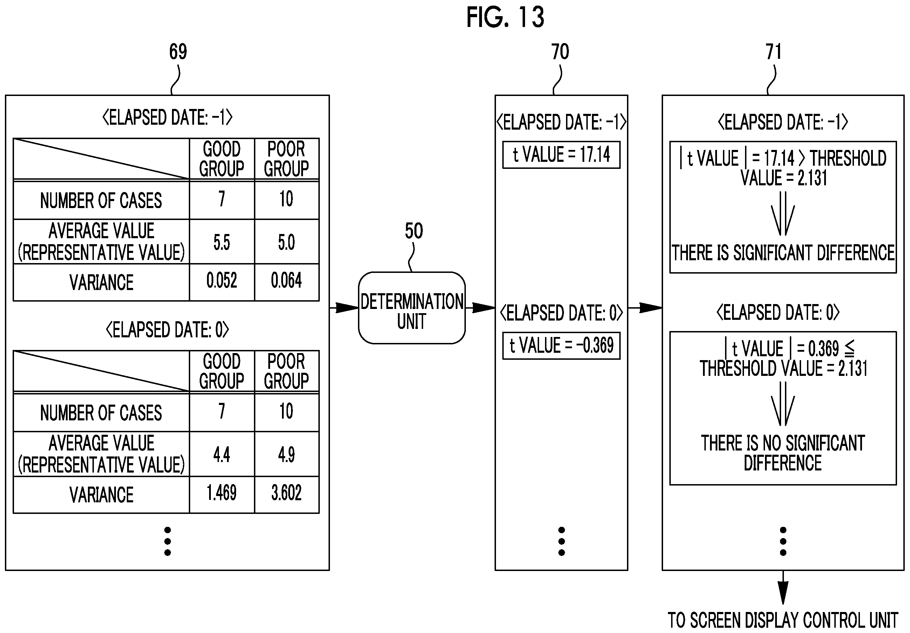

In Fig. 13, the determination unit 50 determines whether or not there is a significant difference between the average values of the examination values of the comparative cases of the good group and the poor group based on the statistical data from the calculation unit 49. More specifically, the determination unit 50 performs determination by t test.

As is known in the field of statistical processing, the t test is an algorithm for determining whether the null hypothesis that there is no significant difference between two groups each having a plurality of numerical values is correct or whether the alternative hypothesis that there is a significant difference between two groups is correct. In the t test, for example, the t value is calculated by Equation (1) having the number of numerical values of each of two groups (number of cases), an average value thereof, and a variance thereof as variables.

$$t = (X1AVE - X2AVE)/\sigma[(1/N1) + (1/N2)\}^{1/2} \cdots (1)$$

X1AVE is the average value of numerical values of one of the two groups, X2AVE is the average value of numerical values of the other group of the two groups, N1 is the number of numerical values of one of the two groups, and N2 is the number of numerical values of the other group of the two groups. $\sigma$ is a variance obtained by combining variances $\sigma1^2$ and $\sigma2^2$ of the numerical values of two groups, and is expressed by Equation (2).

$$\sigma = \{\sigma1^2(N1 - 1) + \sigma2^2(N2 - 1)/(N1 + N2 - 2)\}^{1/2} \cdots (2)$$

The determination unit 50 compares the absolute value of the calculated t value with a threshold value derived statistically and probabilistically. Then, in a case where the absolute value of the t value is larger than the threshold value, it is determined that the alternative hypothesis is correct, that is, there is a significant difference between the two groups. In a case where the absolute value of the t value is equal to or less than the threshold value, it is determined that the null hypothesis is correct, that is, there is no significant difference between the two groups.

Fig. 13 shows how to determine whether or not there is a significant difference between the average values of the examination values of comparative cases of a good group and a poor group by performing t test for each elapsed date for the ALB examination value. For example, in the case of elapsed date "-1", X1AVE = 5.5, X2AVE = 5.0, N1 = 7, N2 = 10, $\sigma1^2$ = 0.052, and $\sigma2^2$ = 0.064. From Equations (1) and (2), t = 17.14 as shown in a t value calculation result 70. In this case, since the absolute value 17.14 of the t value is larger than the threshold value 2.131, there is a significant difference in the determination as shown in a determination result 71. On the other hand, in the case of elapsed date "0", t = -0.369, and the absolute value 0.369 is equal to or less than the threshold value 2.131. Accordingly, there is no significant difference in the determination.

In a case where there is no significant difference in all determinations on all elapsed dates, the determination unit 50 determines that there is no significant difference between the good group and the poor group. On the other hand, as exemplified in Fig. 13, in a case where it is determined that there is a significant difference even on one day of the elapsed dates, it is determined that there is a significant difference between the good group and the poor group.

In Fig. 14, the medical examination data display screen 20 has a medical examination data display region 76. A display field 77 of major category names of medical examination data, such as therapeutic drug, vital signs, and subject examinations, and names of individual items, such as therapeutic drug A, blood pressure (maximum), body temperature, WBC, and ALB, is disposed on the vertical axis of the medical examination data display region 76. A display field 78 of the acquisition period of medical examination data displayed in the medical examination data display region 76 is disposed on the horizontal axis of the medical examination data display region 76.

The display field 78 is divided into a first display field 78A and a second display field 78B. The time scale of a period expressed in the first display field 78A (referred to as a first period) is relatively longer than that of a period expressed in the second display field 78B (referred to as a second period).

A period indicator 79 is provided in the first display field 78A. The period indicator 79 shows to which part of the first period the second period corresponds. The width of the period indicator 79 corresponds to the width of the second period in the time scale of the first period. In Fig. 14, since the second period is about three and a half months from December, 2014 to mid-March, 2015, the width of the period indicator 79 corresponds to the width of about three and a half months in the time scale of the first period.

By moving the period indicator 79 in the horizontal direction with a cursor 80 or by changing the width of the period indicator 79, it is possible to change the display range of the second period. The second period to be first displayed on the medical examination data display screen 20 may be a period before a predetermined period from the latest medical examination data, or may be designated by the medical staff in the case of inputting the patient ID of the medical examination target patient by distribution instruction on the initial screen on the web browser.

The medical examination data display region 76 is divided into a plurality of sub-regions 81A, 81B, and 81C for each major category of medical examination data. Therapeutic drug, vital signs, and subject examinations are assigned to the sub-regions 81A, 81B, and 81C, respectively. A scroll bar 82 for displaying a non-display item by vertical scroll operation is provided in the display field 77 of the sub-regions 81A to 81C.

In the sub-region 81A, administration start and end dates of therapeutic drugs A and B in the second period and a bar 83 indicating the dose are displayed. In the sub-regions 81B and 81C, a line graph 84 obtained by plotting the examination values of vital signs and subject examinations in the second period for each acquisition date and connecting the examination values with a line is displayed. In the display field 77 of vital signs and subject examinations, the legend of the line graph 84 is displayed.

The bar 83 displayed in each of the sub-regions 81A to 81C and the points forming the line graph 84 are disposed at the positions of the medical examination data display region 76 corresponding to the administration date, the measurement date, and the examination date.

In addition to the medical examination data display region 76, a patient information display region 85 or a disease name display region 86 is provided on the medical examination data display screen 20. Character information indicating patient ID and name, date of birth, and age of a medical examination target patient is displayed on the patient information display region 85. Character information indicating a diagnostic disease name, such as "lung cancer", is displayed in the disease name display region 86.

A check box 87 is provided next to the item of each examination value in the display field 77. A case search button 88 is provided below the medical examination data display region 76. The check box 87 and the case search button 88 are for inputting an instruction to distribute the graph comparison display screen 21. In a case where the check box 87 of the item of a desired examination value is selected by the cursor 80 and the case search button 88 is selected by the cursor 80, the browser control unit 36 issues a second distribution request, which includes the item of the examination value, for which the check box 87 has been selected, to the medical examination assistance server 11. Fig. 14 shows how the check box 87 of the ALB item is selected.

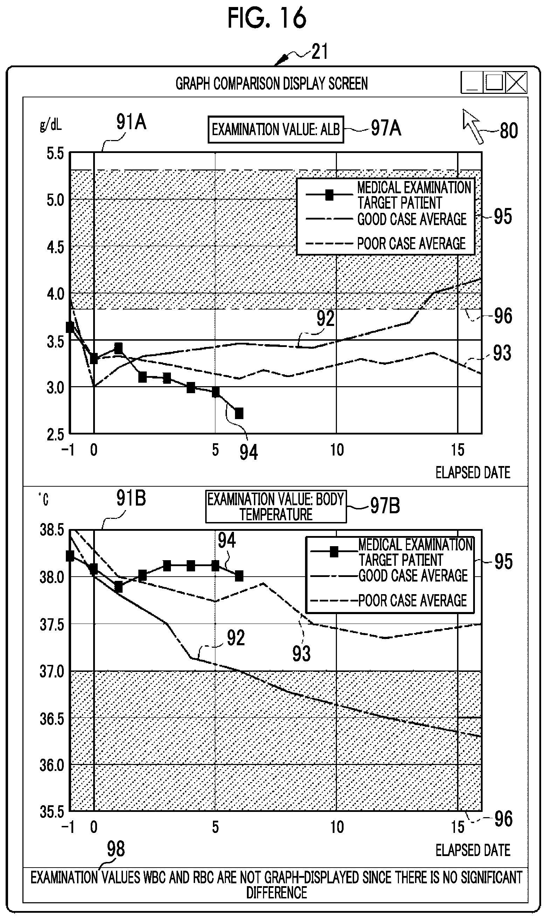

In a case where a plurality of check boxes 87 are selected and the case search button 88 is selected, the calculation unit 49 calculates a representative value for each of a plurality of items. In addition, the determination unit 50 performs determination ermines for each of a plurality of items. The screen display control unit 51 generates the graph comparison display screen 21 including a representative value of an item, which is determined that there is a significant difference by the determination unit 50, among a plurality of items. A representative value of an item determined that there is no significant difference by the determination unit 50 is not displayed on the graph comparison display screen 21 (refer to Fig. 16).

The graph comparison display screen 21 is pop-up displayed, for example, on the medical examination data display screen 20. In Fig. 15, the graph comparison display screen 21 has a graph display region 91. The examination value is assigned to the vertical axis of the graph display region 91, and the elapsed date is assigned to the horizontal axis.

A line graph 92 showing a time-series change in the representative value of a good group shown by the one-dot chain line, a line graph 93 showing a time-series change in the representative value of a poor group shown by the broken line, and a line graph 94 showing a time-series change in the examination value of the medical examination target patient shown by the solid line and square dots are displayed so as to overlap each other in the graph display region 91. These graphs 92 to 94 are displayed along the time axis with the elapsed date "0" as the reference date.

In addition to the graphs 92 to 94, a legend 95 and a normal range 96 of the examination value are displayed in the graph display region 91. As shown by hatching and two-dot chain line, the normal range 96 is displayed so as to be distinguished from the abnormal range and is displayed in a band shape over the entire horizontal axis of the graph display region 91.

In addition to the graph display region 91, an item display region 97 is provided on the graph comparison display screen 21. Character information indicating items of examination values is displayed in the item display region 97.

Fig. 15 shows an example in which the graphs 92 to 94 are displayed for the ALB examination value. In this case, the unit on the vertical axis is "g/dL", and the elapsed date on the horizontal axis is a period before and after the reference date from the elapsed date "-1" before the elapsed date "0", which is the reference date, to the elapsed date "16", for example. The normal range 96 is displayed in the range of 3.8 g/dL to 5.3 g/dL, which is regarded as the normal range of ALB. The elapsed date on the horizontal axis can be changed, for example, from the elapsed date "-10" to the elapsed date "20".

Fig. 15 exemplifies a case in which the graph comparison display screen 21 is displayed by outputting the second distribution request in a case where six days has passed from the reference date for treatment for the medical examination target patient. Therefore, the line graph 94 showing a time-series change in the examination value of the medical examination target patient is discontinued after the elapsed date "6" since there is no examination value after the elapsed date "6".

Fig. 16 is an example of the graph comparison display screen 21 in a case where the second distribution request including the four items of body temperature, WBC, RBC, and ALB is received by the reception unit 46. Here, a case is shown in which the determination unit 50 determines that there is a significant difference in body temperature and ALB, among the four items described above, and the determination unit 50 determines that there is no significant difference in WBC and RBC. In this case, on the graph comparison display screen 21, two graph display regions 91A and 91B for ALB and two item display regions 97A and 97B for body temperature are provided, and a display field 98 of character information indicating items determined that there is no significant difference by the determination unit 50 is provided below the two graph display regions 91A and 91B and the two item display regions 97A and 97B. In the graph display region 91B of body temperature, the normal range 96 is displayed in the range of 35.5°C to 37.0°C, which is regarded as the normal range of body temperature.

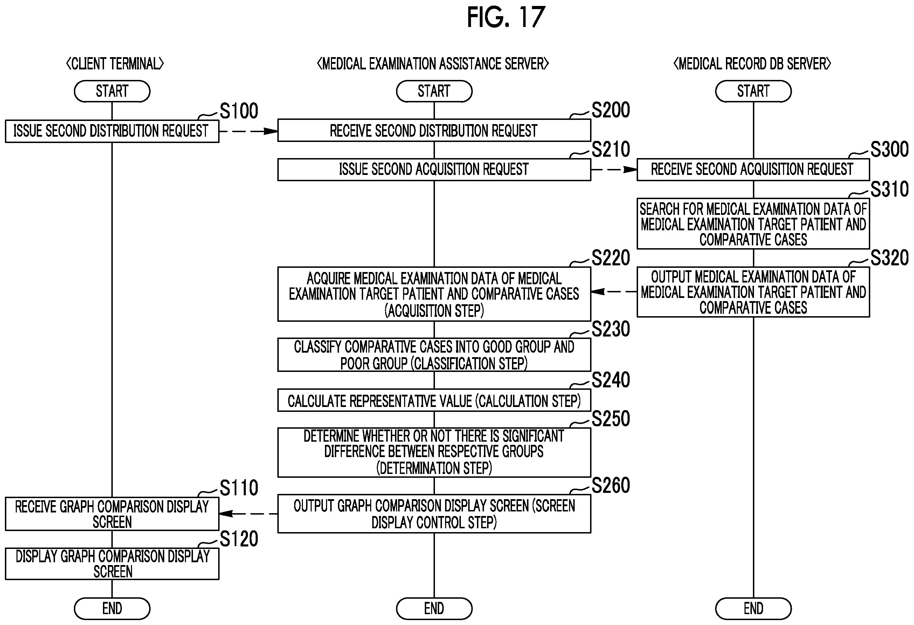

Hereinafter, the operation of the above configuration will be described with reference to the flowchart shown in Fig. 17. First, a medical staff operates the client terminal 10 to input an instruction to distribute the medical examination data display screen 20 on the initial screen on the web browser. In response to the distribution instruction, a first distribution request is issued from the browser control unit 36 to the medical examination assistance server 11.

In the medical examination assistance server 11, the first distribution request is received by the reception unit 46. The patient ID of the medical examination target patient received by the first distribution request is output to the acquisition unit 47, and the first acquisition request is issued from the acquisition unit 47 to the medical record DB server 12.

In the medical record DB server 12, the first acquisition request is received by the reception unit 56. The patient ID of the medical examination target patient received by the first acquisition request is output to the search unit 57. Then, the search unit 57 searches for the medical examination data of the medical examination target patient from the electronic medical record DB 14. The searched medical examination data of the medical examination target patient is output to the medical examination assistance server 11 by the output control unit 58.

In the medical examination assistance server 11, the medical examination data of the medical examination target patient is acquired by the acquisition unit 47. The medical examination data of the medical examination target patient is output to the screen display control unit 51. In the screen display control unit 51, the medical examination data display screen 20 is generated based on the medical examination data of the medical examination target patient. The XML data of the medical examination data display screen 20 is output to the client terminal 10 that is the output source of the first distribution request.

In the client terminal 10, the XML data of the medical examination data display screen 20 is received by the browser control unit 36. The medical examination data display screen 20 to be displayed on the web browser is reproduced by the browser control unit 36 based on the XML data, and the medical examination data display screen 20 is displayed on the display 29A by the GUI control unit 35.

Various kinds of medical examination data of the medical examination target patient are displayed in time series on the medical examination data display screen 20. The medical staff browses the medical examination data display screen 20 to check the medical condition of the medical examination target patient.

On the medical examination data display screen 20, in order to check the medical condition of the medical examination target patient in more detail, the medical staff selects the check box 87 of the item of the desired examination value with the cursor 80 and selects the case search button 88 with the cursor 80. In response to the instruction to distribute the graph comparison display screen 21, as shown in step S100 of Fig. 17, the second distribution request including the item of the examination value for which the check box 87 has been selected is issued from the browser control unit 36 to the medical examination assistance server 11.

In the medical examination assistance server 11, the second distribution request is received by the reception unit 46 (step S200). The patient ID of the medical examination target patient received by the second distribution request is output to the acquisition unit 47, and the item of the examination value is output to the calculation unit 49. The second acquisition request is issued from the acquisition unit 47 to the medical record DB server 12 (step S210).

In the medical record DB server 12, the second acquisition request is received by the reception unit 56 (step S300). The patient ID of the medical examination target patient received by the first-time second acquisition request and the CPID of the medical examination target patient received by the second-time second acquisition request are output to the search unit 57. Then, the search unit 57 searches for the medical examination data of the medical examination target patient from the electronic medical record DB 14 in response to the first-time second acquisition request, and searches for comparative cases in response to the second-time second acquisition request (step S310). These are output to the medical examination assistance server 11 by the output control unit 58 (step S320).

In the medical examination assistance server 11, the acquisition unit 47 acquires the medical examination data of the medical examination target patient and the comparative cases (step S220; acquisition step). The comparative cases acquired at this time are medical examination data having the same CP as the medical examination target patient.

Since the CP is generated for each disease type or disease name, the comparative cases are medical examination data of patients having at least the same disease type or disease name as the medical examination target patient. In addition, since the CP is a summary of treatment plan of each day, the comparative cases have the same item or acquisition date of examination values as the medical examination data of the medical examination target patient unless variance occurs. Therefore, it is possible to acquire comparative cases that are easy to be compared with the medical examination data of the medical examination target patient.

The acquisition unit 47 outputs the acquired medical examination data of the medical examination target patient to the screen display control unit 51, and outputs the acquired comparative cases to the classification unit 48 and the calculation unit 49.

Then, the classification unit 48 classifies the comparative cases into a good group and a poor group according to the length of the treatment period and whether the patient is alive or dead (step S230; classification step). Specifically, first, for each of the comparative cases, the treatment period is calculated based on the application start date and discharge date of the CP recorded in the medical examination and treatment records. Then, the treatment period is compared with a threshold value. In a case where the treatment period is less than the threshold value, the comparative case is classified into the good group. In a case where the treatment period is equal to or greater than the threshold value or the patient is dead, the comparative case is classified into the poor group. The classification result is output to the calculation unit 49.

Then, the calculation unit 49 calculates a representative value representing the group based on the examination values of the comparative cases belonging to the same group (step S 240; calculation step). The statistical data 69 including the number of examination values of the comparative cases of each of the good group and the poor group, the average value (representative value) of the examination values, and the variance of the examination values is calculated. At this time, the application start date of the CP is set as the reference date. As the representative value, an average value of examination values of items, which are included in the second distribution request, for each elapsed date is calculated. The calculation result is output to the determination unit 50 and the screen display control unit 51.

In step S250, based on the statistical data 69, it is determined whether or not there is a significant difference between the good group and the poor group using t test (determination step). This determination is performed for each item included in the second distribution request. The determination result is output to the screen display control unit 51.

The screen display control unit 51 generates the graph comparison display screen 21 based on the medical examination data of the medical examination target patient and the representative value of the item, which is determined that there is a significant difference by the determination unit 50, among the representative values of the respective items. The XML data of the graph comparison display screen 21 is output to the client terminal 10 that is the output source of the second distribution request (step S260; screen display control step).

In the client terminal 10, the XML data of the graph comparison display screen 21 is received by the browser control unit 36 (step S110). The graph comparison display screen 21 to be displayed on the web browser is reproduced by the browser control unit 36 based on the XML data, and the graph comparison display screen 21 is displayed on the display 29A by the GUI control unit 35 (step S120).

The line graph 92 showing a time-series change in the representative value of the good group, the line graph 93 showing a time-series change in the representative value of the poor group, and the line graph 94 showing a time-series change in the examination value of the medical examination target patient are displayed on the graph comparison display screen 21 so as to overlap each other along the time axis with the elapsed date "0" as a reference. The normal range 96 is displayed on the graph comparison display screen 21. The graphs 92 to 94 up to the elapsed date (elapsed date "16" in Fig. 15 or the like) after the elapsed date "0", which is a reference date, are displayed with the elapsed date (elapsed date "-1" in Fig. 15 or the like) before the elapsed date "0" as a starting point.

According to the graph comparison display screen 21, time-series changes in the representative value the good group, the representative value of the poor group, and the examination value of the medical examination target patient can be known at a glance. Therefore, even an inexperienced doctor can easily and reliably ascertain the sign of deterioration of the medical condition of the medical examination target patient. Since the normal range 96 is displayed, it can also be seen how much the examination value of the medical examination target patient and the like are different from the normal range 96. This helps to determine the future medical condition of the medical examination target patient. Since the graphs 92 to 94 in a period before and after the elapsed date "0" that is the reference date are displayed, it is possible to check the medical condition of the patient from the reference date. Therefore, it is easy to check the treatment effect.

Since the application start date of the CP is set as the reference date by the calculation unit 49, the graphs 92 to 94 on the graph comparison display screen 21 can be displayed so as to overlap each other along the time axis. As a result, since it becomes easy to compare the graphs 92 to 94, it also becomes easy to determine the future medical condition of the medical examination target patient.

On the graph comparison display screen 21 shown in Fig. 15, the representative value of the good group tends to increase after the elapsed date "0", while the representative value of the poor group tends to gradually decrease from the elapsed date "0" to the elapsed date "6". After the elapsed date "2", the examination value of the medical examination target patient is lower than the representative value of the poor group. For this reason, since it is possible to predict that the future medical condition of the medical examination target patient will become worse, it is possible to perform appropriate treatment for the medical examination target patient earlier. By performing appropriate treatment for the medical examination target patient earlier, it is possible to contribute to improving the treatment outcome. Therefore, a very good result can be obtained for the medical examination target patient.

Conversely to the case shown in Fig. 15, in a case where the time-series change in the examination value of the medical examination target patient follows the time-series change in the representative value of the good group or is beyond the time-series change in the representative value of the good group, it is possible to predict that the future medical condition of the medical examination target patient will become better.

In the case of the examination value of the item determined that there is a significant difference by the determination unit 50, the difference due to good or bad treatment outcome is obvious. Therefore, the representative value is a useful index for determining the future medical condition of the medical examination target patient. Conversely, in the case of the examination value of the item determined that there is no significant difference by the determination unit 50, there is almost no difference due to good or bad treatment outcome. Therefore, the representative value is not a useful index for determining the future medical condition of the medical examination target patient.

Accordingly, even if the representative value of the item determined that there is no significant difference by the determination unit 50 is displayed so as to be comparable with the examination value of the medical examination target patient, this is troublesome and meaningless since the representative value of the item determined that there is no significant difference does not contribute to the determination of the future medical condition of the medical examination target patient. In the present embodiment, therefore, the determination unit 50 determines whether or not there is a significant difference between the good group and the poor group for each item, and only the representative value of the item determined that there is a significant difference is displayed so as to be comparable with the examination value of the medical examination target patient. In this manner, only useful indices contributing to the determination of the future medical condition of the medical examination target patient can be carefully selected and be provided to the medical staff.

In the first embodiment described above, the average value is calculated as a representative value. However, instead of the average value, a median or a mode may be calculated as a representative value.

The acquisition dates of examination values of respective patients may be different. For example, there is a case where the ALB examination value is acquired after 10 days from the reference date in the case of a patient A while no ALB examination value is acquired due to occurrence of variance after 10 days from the reference date and the ALB examination value is acquired after 11 days from the reference date shifted by one day in the case of a patient B. In such a case, if the elapsed date from the reference date is relatively long, for example,10 days, it is assumed that the difference in acquisition date of about one day is within the allowable range, and the acquisition date is treated as the same date in the case of calculating the representative value.

In the first embodiment described above, determination is performed by t test. However, the present invention is not limited thereto. The determination may be simply performed based on the difference between the representative values of the good group and the poor group. For example, it is determined that there is no significant difference in a case where the difference between the representative values of the good group and the poor group is less than a predetermined threshold value and there is a significant difference in a case where the difference between the representative values of the good group and the poor group is equal to or greater than the threshold value.

In the first embodiment described above, comparative cases are classified by comparing the calculated treatment period with the threshold value. However, comparative cases can be classified according to whether the calculated treatment period is relatively long or short without using a threshold value. Specifically, statistical analysis of the calculated treatment period is performed, and classification is performed based on the frequency distribution with the treatment period as the class on the horizontal axis and the number of comparative cases as the frequency on the vertical axis. For example, comparative cases are classified according to the percentage with respect to all comparative cases, such as classifying 20% of all comparative cases with the shorter treatment period into the good group and classifying 20% of all comparative cases with the longer treatment period into the poor group. In this case, it is preferable to determine the percentage so that a significant difference can be predicted in advance. Alternatively, based on the mode or median of the treatment period, a group having a treatment period shorter than the mode or the median may be classified into the good group and a group having a treatment period longer than the mode or the median may be classified into a poor group. In addition, any method of classifying comparative cases may be used.

In a case where the representative value of each of the good group and the poor group calculated by the calculation unit 49 is in the normal range set for each item of the examination value, even a case where the determination unit 50 determines that there is a significant difference is treated in the same manner as in a case where the determination unit 50 determines that there is no significant difference. Accordingly, it is preferable not to display the respective graphs 92 and 93 for the examination value of the item.

In the first embodiment described above, acquisition and classification of comparative cases, calculation of representative values, and determination of significant difference are performed after receiving the second distribution request. However, these processes may be performed before receiving the second distribution request. Instead of the items included in the second distribution request, calculation of representative values and determination of significant difference may be performed for all items of examination values. In this case, the calculation of representative values and the determination of significant difference are performed in the case of receiving the first distribution request. Then, in the display field 77 of the medical examination data display screen 20, only items determined that there is a significant difference are configured so as to be selectable by the cursor 80, and the graph comparison display screen 21 is displayed in a case where the item is selected by the cursor 80.

In the first embodiment described above, the medical examination data display screen 20 and the graph comparison display screen 21 are set as separate display screens, and the graphs 92 and 93 are displayed separately from the medical examination data display screen 20. However, the graphs 92 and 93 may be displayed on the medical examination data display screen 20. In this manner, since time-series changes in other items can also be viewed on the same display screen, comparison with the other items becomes easy. On the other hand, in a case where the determination unit 50 determines that there are a relatively large number of items determined that there is a significant difference, the number of graphs increases and it becomes difficult to see the graphs in the case of displaying the graphs 92 and 93 on the medical examination data display screen 20. For this reason, it is preferable to display the graphs 92 and 93 separately from the medical examination data display screen 20 as in the first embodiment. A display mode in which the medical examination data display screen 20 and the graph comparison display screen 21 are set as separate display screens and a display mode in which the graphs 92 and 93 are displayed on the medical examination data display screen 20 can be switched.

Alternatively, in the case of displaying the graphs 92 and 93 on the medical examination data display screen 20, a display switching button for switching display and non-display of each of the graphs 92 and 93 may be provided on the medical examination data display screen 20, so that the display and non-display of each of the graphs 92 and 93 is switched according to the operation of the display switching button. In a case where there are a plurality of items determined that there is a significant difference by the determination unit 50, display and non-display of each of the graphs 92 and 93 may be switched for each item.

As a display form of the representative value and the examination value of the medical examination target patient, the graphs 92 to 94 may be displayed in parallel instead of or in addition to the overlapping display of the graphs 92 to 94 of the first embodiment described above. In this case, the graphs 92 to 94 may be displayed in parallel on the same display screen, or may be displayed on different display screens. Instead of the form of a line graph, representative values and examination values of the medical examination target patient may be displayed in the form of a table. In short, representative values and examination values of the medical examination target patient may be displayed in a comparable manner.

[Second embodiment]

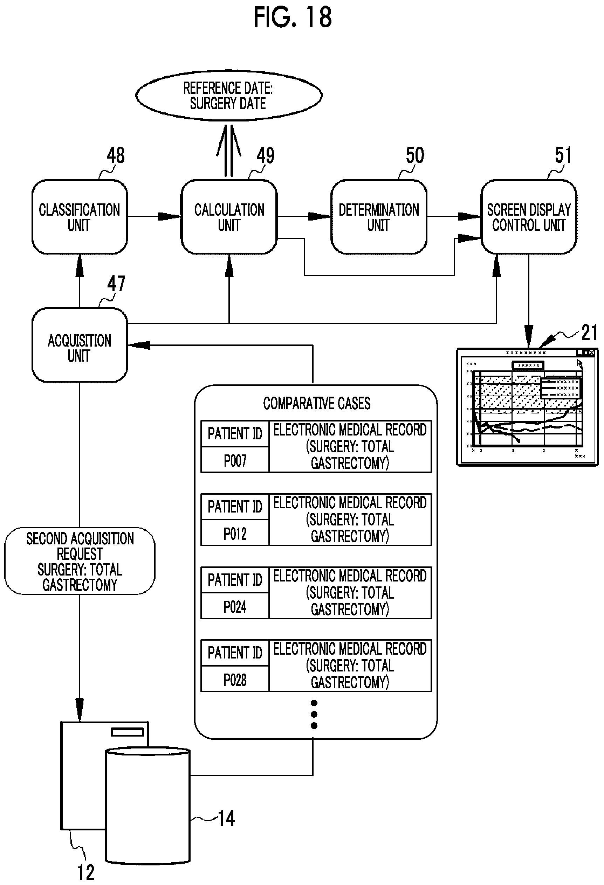

In the first embodiment described above, medical examination data having the same CP as the medical examination target patient is acquired as a comparative case. However, in a second embodiment, as shown in Fig. 18, medical examination data having the same surgical contents as the medical examination target patient is acquired as a comparative case.

Fig. 18 exemplifies a case in which the surgical contents "total gastrectomy" are recorded in the examination and treatment record of the electronic medical record of the medical examination target patient. In this case, the acquisition unit 47 outputs a second acquisition request including the surgical contents "total gastrectomy" to the medical record DB server 12. In the medical record DB server 12, the search unit 57 searches for the medical examination data of the electronic medical record of patients (patients with patient IDs "P007", "P012", and the like) in which the surgical contents "total gastrectomy" are recorded in the medical examination and treatment record, as a comparative case, from the electronic medical record of each patient of the electronic medical record DB 14.

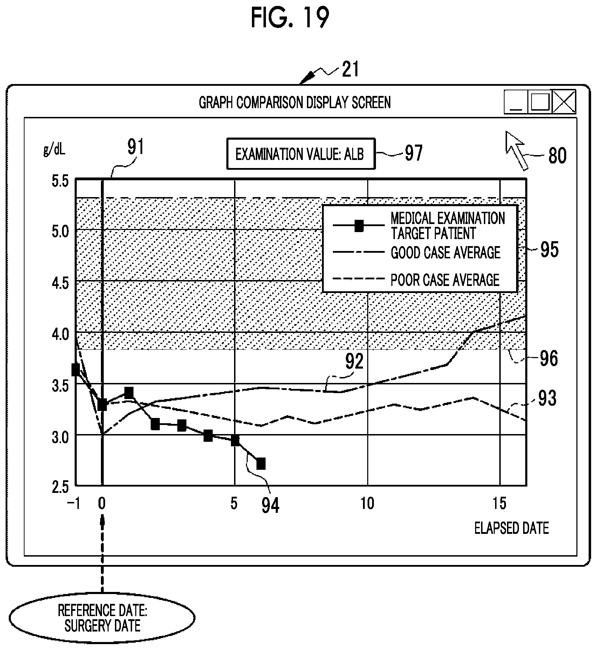

In this case, the surgery date is set as a reference date by the calculation unit 49. Therefore, as shown in Fig. 19, the graphs 92 to 94 on the graph comparison display screen 21 are displayed along the time axis with the surgery date as the elapsed date "0".

The surgical contents are information indirectly indicating the disease type or disease name. For example, in a case where the surgical contents are "total gastrectomy" in Fig. 18, it can be seen that the disease name is gastric cancer. In a case where the surgical contents are "right hepatic lobectomy", it can be seen that the disease name is liver cancer. Accordingly, as in the case of the first embodiment described above, comparative cases acquired by the acquisition unit 47 are medical examination data of patients having at least the same disease type or disease name as the medical examination target patient. If the disease type or the disease name is the same, the items of examination values are also approximately the same between comparative cases and the medical examination data of the medical examination target patient. Therefore, it is possible to acquire comparative cases that are easy to be compared with the medical examination data of the medical examination target patient.

As the surgical contents, the contents of the procedure, such as laparotomy, endoscopic mucosal resection (EMR), endoscopic submucosal dissection (ESD), and laparoscopy and endoscopy cooperative surgery (LECS), may be included.

[Third embodiment]

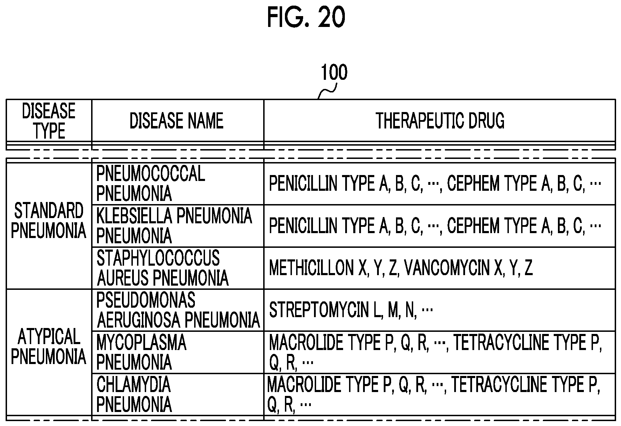

In a third embodiment, medical examination data having the same disease name and therapeutic drug as a medical examination target patient is acquired as a comparative case. In this case, a disease name and therapeutic drug table 100 shown in Fig. 20 is recorded in the storage device 25B of the medical examination assistance server 11.

In Fig. 20, in the disease name and therapeutic drug table 100, the disease name is classified for each disease type, and a collection of therapeutic drugs are shown for each disease name. Fig. 20 shows an example in which the disease type of pneumonia is classified into "standard pneumonia" and "atypical pneumonia", disease names "pneumococcal pneumonia", "Klebsiella pneumonia pneumonia", and "Staphylococcus aureus pneumonia" are classified into "standard pneumonia", and disease names "pseudomonas aeruginosa pneumonia", "mycoplasma pneumonia", and "chlamydia pneumonia" are classified into "atypical pneumonia". Also for diseases other than the pneumonia shown in the diagram, disease names and their therapeutic drugs classified according to the disease type are registered in the disease name and therapeutic drug table 100. For example, in a case where the disease is hepatitis, the disease type is classified into "viral hepatitis" and "other hepatitis", "hepatitis A", "hepatitis B", and the like are classified into "viral hepatitis", and "alcoholic hepatitis", "autoimmune hepatitis", and the like are classified into "other hepatitis".

"Pneumococcal pneumonia" and "Klebsiella pneumonia pneumonia" of "standard pneumonia" have the same therapeutic drugs. In addition, "mycoplasma pneumonia" and "chlamydia pneumonia" of "atypical pneumonia" have the same herapeutic drugs. Therapeutic drugs "penicillin type A, B, C, ... ", "macrolide type P, Q, R, ... ", and the like have almost the same ingredients and efficacy except only that the pharmaceutical companies are different, for example. The acquisition unit 47 regards disease names having the same disease type and therapeutic drugs as the same type of disease names, and regards the therapeutic drugs as the same type of therapeutic drugs.

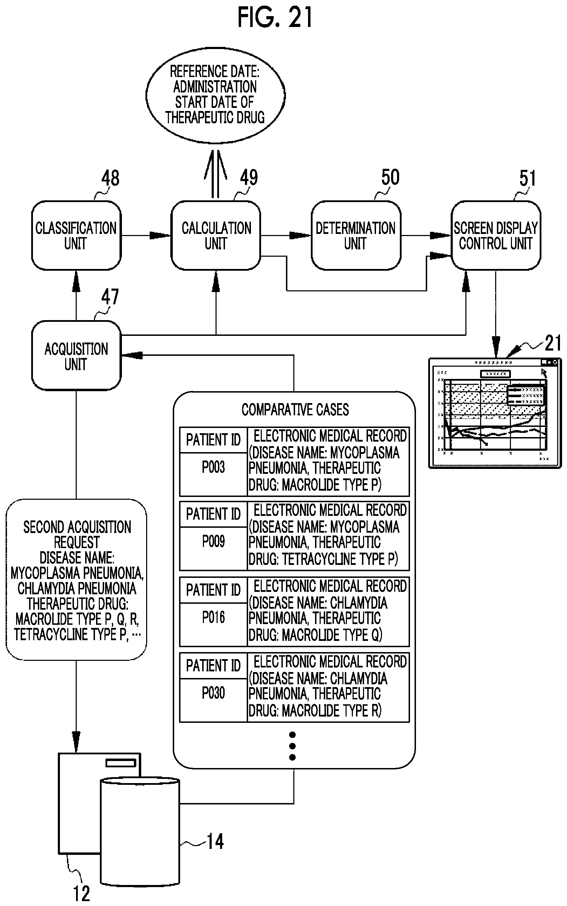

As shown in Fig. 21, in a case where the disease name "mycoplasma pneumonia" and the therapeutic drug "macrolide type P" are recorded in the medical examination and treatment record of the electronic medical record of the medical examination target patient, the acquisition unit 47 outputs a second acquisition request, which includes the disease name "mycoplasma pneumonia" and the therapeutic drug "macrolide type P" and the disease name ("chlamydia pneumonia") and the therapeutic drugs ("tetracycline type P", "macrolide type Q", "macrolide type R", and the like) of the same type as the disease name "mycoplasma pneumonia" and the therapeutic drug "macrolide type P", to the medical record DB server 12 with reference to the disease name and therapeutic drug table 100.

In the medical record DB server 12, the search unit 57 searches for medical examination data of the electronic medical record of patients (patients with patient IDs "P003", "P009", and the like), in which the disease name "mycoplasma pneumonia" or the disease name "chlamydia pneumonia" of the same type as the disease name "mycoplasma pneumonia" and the therapeutic drug "macrolide type P" or the therapeutic drugs "tetracycline type P", "macrolide type Q", "macrolide type R", and the like of the same type as the therapeutic drug "macrolide type P" are recorded in the medical examination and treatment record, as a comparative case. That is, medical examination data having the same disease name and therapeutic drugs as the medical examination target patient includes medical examination data having completely the same disease name and therapeutic drugs as the medical examination target patient and medical examination data having the same type of disease name and therapeutic drugs as the medical examination target patient.

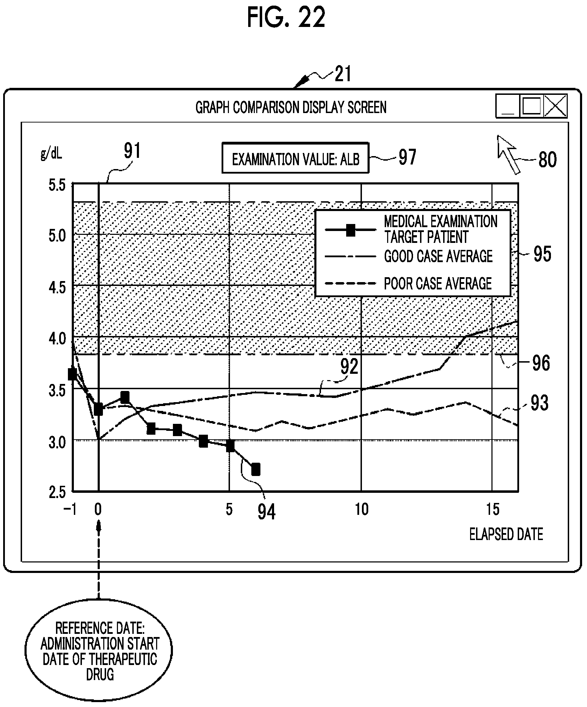

In this case, the administration start date of the therapeutic drug is set as the reference date by the calculation unit 49. Therefore, as shown in Fig. 22, the graphs 92 to 94 on the graph comparison display screen 21 are displayed along the time axis with the administration start date of the therapeutic drug as the elapsed date "0".

Comparative cases in this case are medical examination data of patients having at least the same disease name and therapeutic drug as the medical examination target patient or patients having the same type of disease name and therapeutic drug as the medical examination target patient. If disease names are the same or the same type, the items of examination values are also approximately the same between comparative cases and the medical examination data of the medical examination target patient. In addition, if therapeutic drugs are the same or the same type, the treatment effect is the same. Therefore, it is possible to acquire comparative cases that are easy to be compared with the medical examination data of the medical examination target patient.

The disease name and therapeutic drug table 100 may be recorded in the storage device 25C of the medical record DB server 12. In this case, only the disease name and the therapeutic drug of the medical examination target patient are included in the second acquisition request. The search unit 57 reads disease names and therapeutic drugs of the same type as the disease name and the therapeutic drug of the medical examination target patient from the disease name and therapeutic drug table 100, and searches for medical examination data of the electronic medical record of patients, in which the disease name and the therapeutic drug of the medical examination target patient of the second acquisition request and the disease names and the therapeutic drugs of the same type as the disease name and the therapeutic drug of the medical examination target patient read from the disease name and therapeutic drug table 100 are recorded in the medical examination and treatment record, as comparative cases.

The methods of acquiring comparative cases in the respective embodiments described above may be used in combination. For example, medical examination data having the same CP and surgical contents as the medical examination target patient is acquired as a comparative case.

[Fourth embodiment]

In the respective embodiments described above, examples are shown in which medical examination data having the same CP as the medical examination target patient (first embodiment), medical examination data having the same surgical contents as the medical examination target patient (second embodiment), and medical examination data having the same disease name and therapeutic drug as the medical examination target patient (third embodiment) are acquired as comparative cases. However, the present invention is not limited thereto.

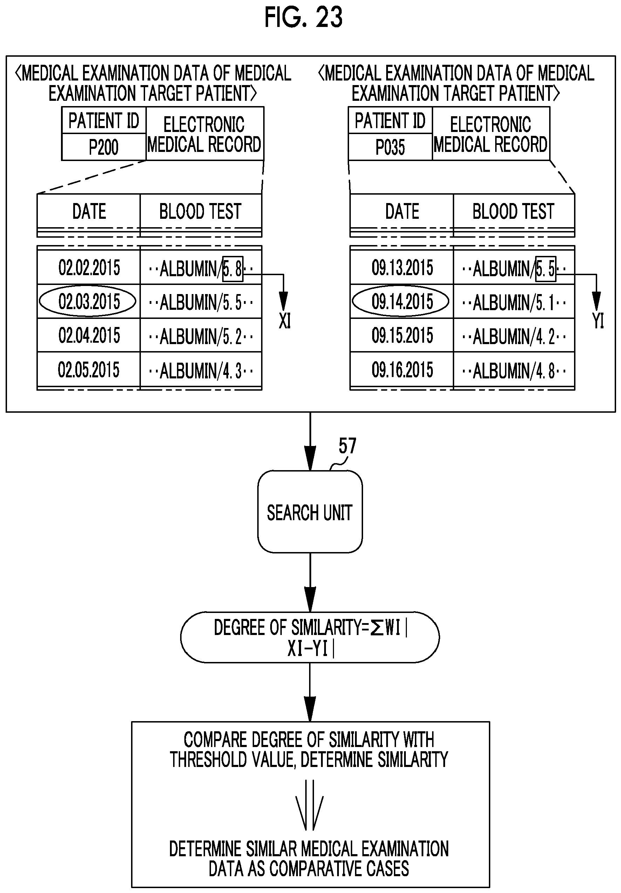

In a fourth embodiment shown in Fig. 23, the search unit 57 calculates the degree of similarity between the examination value of the medical examination target patient and the examination value of a patient other than the medical examination target patient. The degree of similarity is calculated based on the examination value of the medical examination target patient and the examination value of a patient other than the medical examination target patient on the acquisition date before the reference date, such as the application start date of the CP, the surgery date, and the administration start date of the therapeutic drug.

For example, the degree of similarity is a sum ($\Sigma WI|XI - YI|$) calculated by taking a difference $|XI - YI|$ between an examination value $XI$ ($I = 1, 2, ..., M$; M is the number of items of examination values) of each item on the acquisition date before the reference date of the medical examination target patient and an examination value $YI$ of each item on the acquisition date before the reference date of a patient other than the medical examination target patient and by multiplying the difference between the examination values of each item by an appropriate weighting coefficient $WI$ (refer to Fig. 23). The degree of similarity calculated in this manner indicates a distance in the M-dimensional vector space of two M-dimensional vectors respectively having $XI$ and $YI$ as elements. In addition, the weighting coefficient $WI$ is set in advance according to the disease name or the like.

As the difference $|XI - YI|$ between the examination value of the medical examination target patient and the examination value of a patient other than the medical examination target patient decreases, the degree of similarity decreases. Accordingly, it can be said that the similarity between the medical examination data of the medical examination target patient and the medical examination data of a patient other than the medical examination target patient is high. On the other hand, as the difference $|XI - YI|$ increases, the degree of similarity increases. Accordingly, it can be said that the similarity between the medical examination data of the medical examination target patient and the medical examination data of a patient other than the medical examination target patient is low.

The search unit 57 compares the calculated degree of similarity with a predetermined threshold value. Then, in a case where the degree of similarity is less than the threshold value, it is determined that the medical examination data of patients other than the medical examination target patient is similar to the medical examination data of the medical examination target patient. On the other hand, in a case where the degree of similarity is equal to or greater than the threshold value, it is determined that the medical examination data of patients other than the medical examination target patient is not similar to the medical examination data of the medical examination target patient. The search unit 57 determines the medical examination data, which is determined that there is a similarity, as a comparative case.

Fig. 23 shows how to determine similarity by calculating the degree of similarity between the medical examination data of a medical examination target patient having a patient ID "P200" and the medical examination data of a patient having a patient ID "P035" other than the medical examination target patient. As the examination values $XI$ and $YI$ on the acquisition date before the reference date, examination values "5.8" and "5.5" before reference dates "2015. 02.03" and "2012. 09.14" surrounded by the solid ellipse are picked up. In Fig. 23, only the ALB examination value is shown. However, also for examination values of other items, examination values before the reference dates are picked up and used for calculation of degree of similarity.

Since the medical examination data as a comparative case is determined according to the degree of similarity between the examination value of the medical examination target patient and the examination value of a patient other than the medical examination target patient, medical examination data more similar to the medical examination data of the medical examination target patient can be determined as a comparative case.

In the first to third embodiments described above, since the similarity between examination values is not taken into consideration, medical examination data with a low similarity with the medical examination data of the medical examination target patient may be acquired as a comparative case. Then, since a difference occurs between the examination value of the medical examination target patient and the representative value calculated by the calculation unit 49, it becomes difficult to compare the graphs 92 to 94 with each other.

In contrast, in the present embodiment, medical examination data more similar to the medical examination data of the medical examination target patient can be determined as a comparative case. Accordingly, since the occurrence of a difference between the examination value of the medical examination target patient and the representative value calculated by the calculation unit 49 is prevented, it becomes easy to compare the graphs 92 to 94 with each other.

In this case, however, it is necessary to calculate the degree of similarity for all pieces of medical examination data of patients other than the medical examination target patient registered in the electronic medical record DB 14. For this reason, in a case where the amount of medical examination data of patients other than the medical examination target patient is relatively large, it takes time to search for and acquire comparative cases. Therefore, after searching for comparative cases first using the method according to any of the first to third embodiments described above, the method of the present embodiment may be applied to further narrow down the searched comparative cases. In this case, the acquisition unit 47 may be responsible for calculation of the degree of similarity or determination of similarity.

In a case where health management information is included in medical examination data, the examination value of the health management information may be used for calculation of degree of similarity.

According to the attributes such as patient's age, sex, and residential area, medical examination data to be searched as a comparative case may be determined, or the searched comparative cases may be narrowed down. In a case where genetic test information is included in the medical examination data, genetic test information may be used to search for or narrow down comparative cases.

[Fifth embodiment]

In the first embodiment described above, comparative cases are classified into the good group and the poor group according to the length of the treatment period. However, the present invention is not limited thereto. In the case of a patient adopting the CP, it is possible to determine whether the treatment outcome is good or bad according to the variance. Therefore, in the fifth embodiment shown in Fig. 24, comparative cases are classified according to the variance.

More specifically, the classification unit 48 of the present embodiment classifies comparative cases, in which no variance is recorded in the medical examination and treatment record, and comparative cases, in which positive variance is recorded in the medical examination and treatment record, into the good group. On the other hand, comparative cases, in which negative variance is recorded in the medical examination and treatment record, and comparative cases, in which the patient is dead, are classified into the poor group.

Here, the positive variance is a variance occurring in a case where the recovery of a patient is better than that assumed in the treatment plan. The negative variance is a variance occurring in a case where the recovery of a patient is not better than that assumed in the treatment plan. Examples of the positive variance include a case where the treatment plan is moved up from those planned and a case where a part of the treatment plan is determined to be unnecessary and is omitted. Examples of the negative variance include a case where the treatment plan is delayed from those planned and a case where complications develop and a treatment plan, which is not included in the original treatment plan, is added.

Fig. 24 shows how to classify comparative cases of patient IDs "P006", "P018", "P021", "P036", and the like into a good group and a poor group. In this case, since no variance is recorded in the medical examination and treatment record of the comparative case of patient ID "P018", the classification unit 48 classifies the comparative case into the good group. In addition, since the positive variance of "revaluation of administration period" is recorded in the medical examination and treatment record of the comparative case of patient ID "P036", the comparative case is classified into the good group. On the other hand, since the negative variance of "extension of dietary restriction" is recorded in the medical examination and treatment record of the comparative case of patient ID "P006" and the negative variance of "addition of blood test" is recorded in the medical examination and treatment record of the comparative case of patient ID "P021", the classification unit 48 classifies the comparative cases into the poor group.

In addition, comparative cases may be classified according to the number of occurrences of positive variance and negative variance. For example, a comparative case is classified into the good group in a case where a result obtained by dividing the number of occurrences of negative variance by the number of occurrences of positive variance is 2 or more, and a comparative case is classified into the poor group in a case where the result is less than 2. Comparative cases may be simply classified based on the presence or absence of negative variance.

Instead of the classification method according to the length of the treatment period in the first embodiment and the variance in the fifth embodiment, comparative cases may be classified according to whether or not the examination value of a specific item is an abnormal value. For example, paying attention to the examination value of body temperature, the comparative case may be classified into the poor group in a case where the examination value of body temperature is equal to or higher than 37.5°C for a certain period of time.

[Sixth embodiment]

Fig. 15 exemplifies the graph comparison display screen 21 in a case where six days has passed from the reference date for treatment for the medical examination target patient. In this case, since there is an obvious difference between the representative value of the good group and the examination value of the medical examination target patient, it is possible to predict that the future medical condition of the medical examination target patient will become worse. Accordingly, it is meaningful to display the graph comparison display screen 21.

However, from the elapsed date "-1" to the elapsed date "2", it cannot be said that there is an obvious difference between the representative value of the good group and the examination value of the medical examination target patient. For this reason, even if the graph comparison display screen 21 is displayed between the elapsed date "-1" to the elapsed date "2", it is difficult to predict the future medical condition of the medical examination target patient. Therefore, while there is no obvious difference between the representative value of the good group and the examination value of the medical examination target patient, it does not make much sense to display the graph comparison display screen 21.

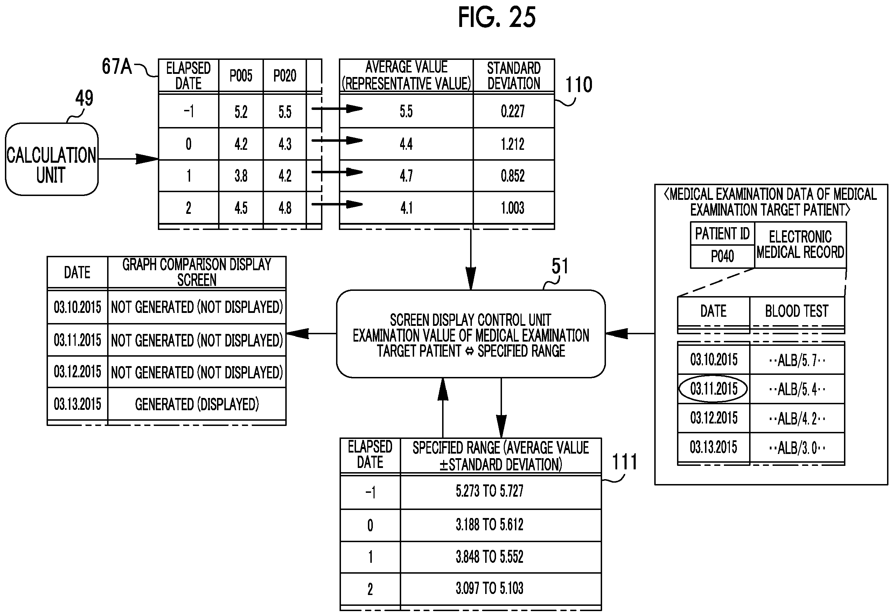

Therefore, in a sixth embodiment shown in Fig. 25, no representative value is displayed while the examination value of the medical examination target patient is within a specified range defined by the examination value of the comparative case classified into the good group, which is a group with the best treatment outcome, by the classification unit 48, and the representative value is displayed in a case where the examination value of the medical examination target patient is outside the specified range.

Specifically, as shown in a calculation result 110 of Fig. 25, the calculation unit 49 calculates a standard deviation, in addition to the average value of the examination values of each elapsed date of the comparative cases of the good group, based on the intermediate processing data 67A of the good group. The screen display control unit 51 generates intermediate processing data 111 based on the calculation result 110. The intermediate processing data 111 defines a specified range for each elapsed date. As the specified range, a numerical range expressed by average value ± standard deviation of each elapsed date of the comparative cases of the good group is set.

The screen display control unit 51 determines whether or not to generate the graph comparison display screen 21 based on the intermediate processing data 111. Specifically, the screen display control unit 51 determines whether or not the examination value of the medical examination target patient from the acquisition unit 47 is within the specified range. The screen display control unit 51 does not generate the graph comparison display screen 21 while the examination value of the medical examination target patient is within the specified range, and generates the graph comparison display screen 21 for the first time in a case where the examination value of the medical examination target patient is outside the specified range.

Fig. 25 exemplifies a case where the ALB examination value of the medical examination data of the medical examination target patient with a patient ID "P040" changes to "5.7, 5.4, 4.2, 3.0, ..." from the date "2015. 03. 10" (elapsed date "-1") before the reference date "2015. 03. 11" (elapsed date "0").

From "2015. 03. 10" corresponding to the elapsed date "-1" to "2015. 03. 12" corresponding to the elapsed date "1", the ALB examination value of the medical examination target patient is within the specified range shown in the intermediate processing data 111. For the first time on "2015. 03. 13" corresponding to the elapsed date "2", the ALB examination value of the medical examination target patient is outside the specified range.

In this case, even if the determination unit 50 determines that there is a significant difference between the ALB examination values from "2015. 03. 10" to "2015. 03. 12", the screen display control unit 51 does not generate the graph comparison display screen 21. Accordingly, the graph comparison display screen 21 is not displayed on the display 29A of the client terminal 10 during this period. On the other hand, in a case where the second distribution request is received on "2015. 03.13", the screen display control unit 51 generates the graph comparison display screen 21. Accordingly, for the first time on "2015. 03.13", the graph comparison display screen 21 is displayed on the display 29A of the client terminal 10.

Thus, since there is an obvious difference between the representative value of the good group and the examination value of the medical examination target patient, the graph comparison display screen 21 is displayed after it becomes possible to predict that the future medical condition of the medical examination target patient will become worse. Accordingly, cases do not occur in which the graph comparison display screen 21, through which it is difficult to predict the future medical condition of the medical examination target patient, is displayed and the medical staff feels confused and troubled.

Instead of the numerical range expressed by average value ± standard deviation exemplified above, the specified range may be a numerical range expressed by average value ± standard deviation × 2 or average value ± standard deviation × 3. In addition, a numerical range that is not based on the standard deviation, such as average value ± 1, may be set as the specified range.

[Seventh embodiment]

In each of the embodiments described above, the representative values are calculated or the graphs 92 to 94 are displayed using the examination values themselves of the medical examination target patient and the comparative cases. However, since there is a difference between the examination values of patients due to individual differences of the patients, there is a possibility that an appropriate representative value cannot be calculated or the graphs 92 to 94 cannot be simply compared using the examination value themselves.

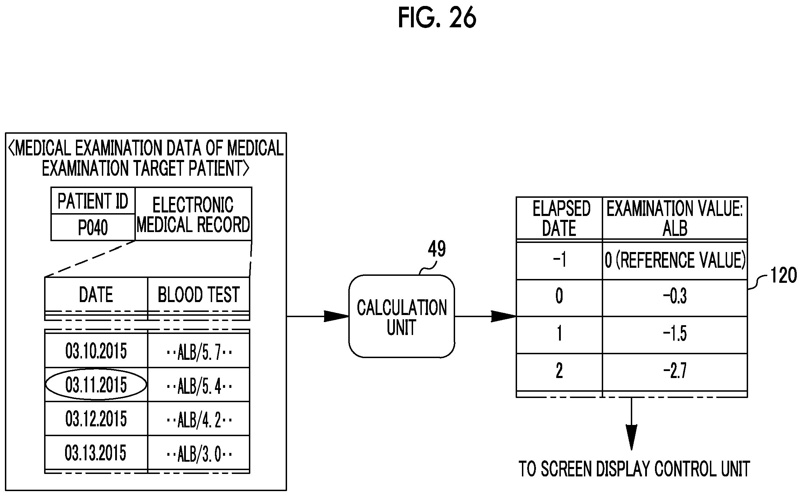

Therefore, in the seventh embodiment shown in Figs. 26 to 28, the examination values of the medical examination target patient and the comparative cases are standardized by the reference value. Specifically, the calculation unit 49 converts the examination values of the medical examination target patient and the comparative cases into the amount of change from the reference value to standardize the examination values of the medical examination target patient and the comparative cases.

Fig. 26 shows how the calculation unit 49 standardizes the ALB examination value of the medical examination target patient with the examination value on the elapsed date "-1" as a reference value. As in the case shown in Fig. 25, the ALB examination value of the medical examination target patient is that of a patient with a patient ID "P040", and changes to "5.7, 5.4, 4.2, 3.0, ...". As shown in a calculation result 120, the calculation unit 49 converts the examination values of "5.7, 5.4, 4.2, 3.0, ..." into the amount of change (difference) "0, -0.3, -1.5, -2.7, ..." from the examination value "5.7" on the elapsed date "-1 ".

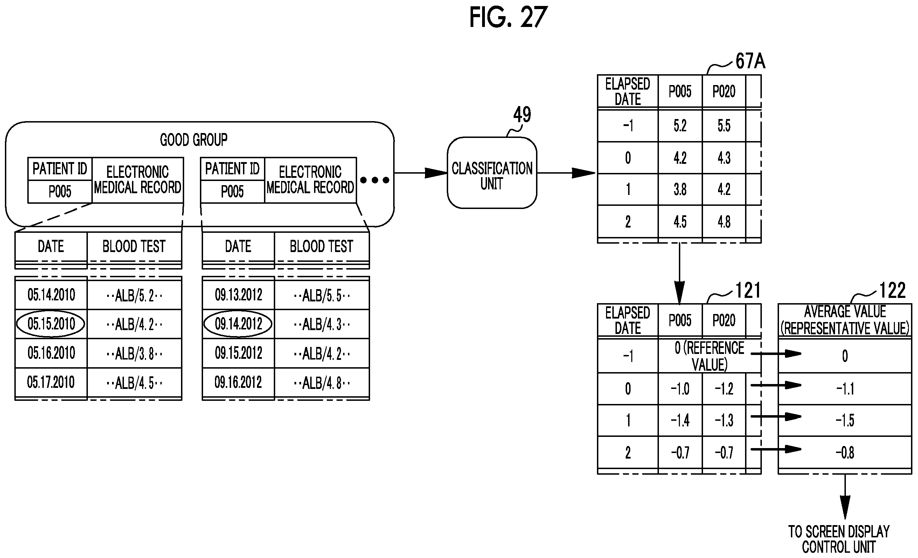

Fig. 27 shows how the calculation unit 49 standardizes the ALB examination values of comparative cases of a good group with the examination value on the elapsed date "-1" as a reference value as in the case shown in Fig. 26. The ALB examination values of the comparative cases of the good group are the same as in the case shown in Fig. 11. In addition, the contents of the intermediate processing data 67A, which is a collection of ALB examination values for each elapsed date of each of comparative cases belonging to the good group, are the same as in the case shown in Fig. 11.

The calculation unit 49 further generates intermediate processing data 121 based on the intermediate processing data 67A. The intermediate processing data 121 is obtained by converting the ALB examination value for each elapsed date of each comparative case belonging to the good group into the amount of change from the examination value on the elapsed date "-1". For example, the ALB examination values of a patient with patient ID "P005" change to "5.2, 4.2, 3.8, 4.5 ..." from the intermediate processing data 67A, but these are converted into the amount of change "0, -1.0, -1.4, -0.7, ..." from the examination value "5.2" on the elapsed date "-1". Although not shown, ALB examination values of comparative cases of the poor group are also converted into the amount of change.

In this case, as shown in a calculation result 122, the calculation unit 49 calculates an average value, which is a representative value, based on the intermediate processing data 121 based on the amount of change instead of the intermediate processing data 67A based on the examination value itself.

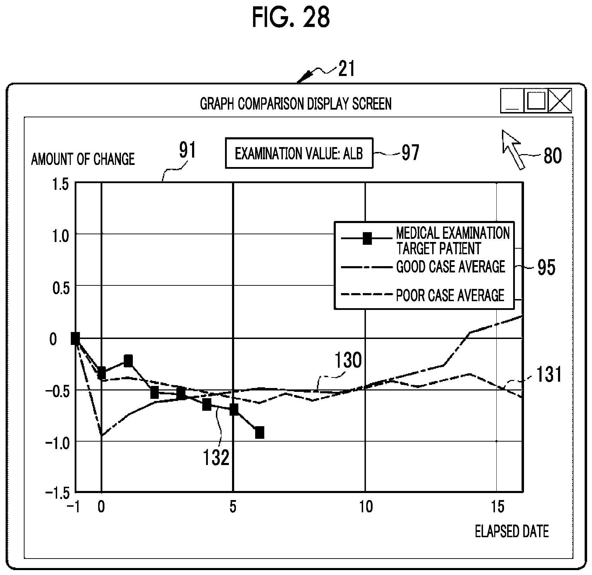

For the amount of change in the examination value of the medical examination target patient and the amount of change in the examination value of the comparative case, the elapsed date "-1" as a reference value is set to "0". Therefore, as shown in Fig. 28, three line graphs 130, 131, and 132 in which the amount of change on the elapsed date "-1" is set to "0" are displayed on the graph comparison display screen 21. The line graph 130 indicated by the one-dot chain line shows a time-series change in the amount of change of the representative value of the good group from the reference value. The line graph 131 indicated by the broken line shows a time-series change in the amount of change of the representative value of the poor group from the reference value. The line graph 132 indicated by the solid line and square points shows a time-series change in the amount of change of the examination value of the medical examination target patient from the reference value. In this case, the amount of change is assigned to the vertical axis of the graph display region 91, and the normal range 96 is not displayed.

Since the examination value of the medical examination target patient and the examination value of the comparative case are standardized by the reference value, it is possible to absorb the difference between examination values due to individual differences of patients. In addition, since the starting points of the graphs 130 to 132 are aligned with the reference value, it becomes easy to compare the graphs 130 to 132 with each other.

Instead of the examination value on the elapsed date "-1 ", the examination value on the elapsed date "0" may be used as the reference value. In a case where health management information is included in medical examination data, the examination value of the health management information may be used as the reference value. On the graph comparison display screen 21, the graphs 92 to 94 based on the examination values themselves shown in Fig. 15 and the graphs 130 to 132 based on the amount of change shown in Fig. 28 may be switched and displayed.

[Eighth embodiment]

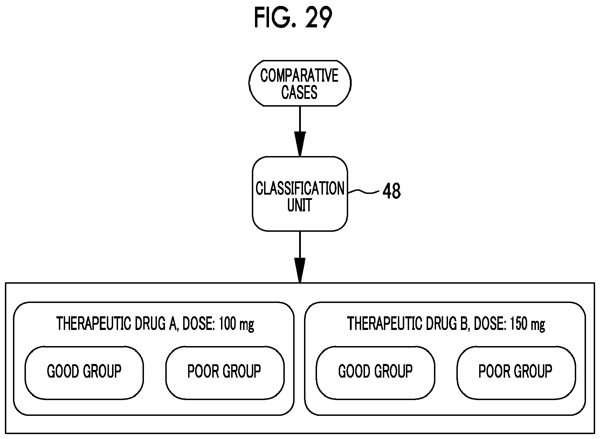

As shown in Fig. 29, in an eighth embodiment, the classification unit 48 further classifies a good group and a poor group according to the type and dose of therapeutic drug registered in the medical examination data. Fig. 29 shows an example in which a good group and a poor group are classified in a case of therapeutic drug A and dose: 100 mg and a case of therapeutic drug B and dose: 150 mg. In addition, the good group and the poor group may be classified for each dose for the same therapeutic drug as in a case of therapeutic drug A and dose: 100 mg and a case of therapeutic drug A and dose: 200 mg.

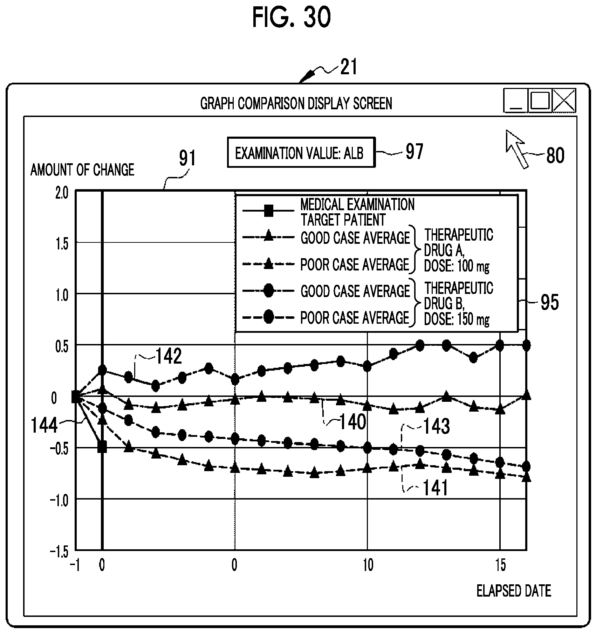

In this case, as shown in Fig. 30, a total of five line graphs 140, 141, 142, 143, and 144 are displayed on the graph comparison display screen 21. The line graphs 140 and 141 indicated by triangular points show a case of therapeutic drug A and dose: 100 mg, the line graphs 142 and 143 indicated by circular points show a case of therapeutic drug B and dose: 150 mg, the line graphs 140 and 142 of the one-dot chain line show a time-series change in the amount of change of the representative value of the good group from the reference value, and the line graphs 141 and 143 of the broken line show a time-series change in the amount of change of the representative value of the poor group from the reference value. The line graph 144 indicated by the solid line and square points shows a time-series change in the amount of change of the examination value of the medical examination target patient from the reference value.

From Fig. 30, it can be seen that the amount of change (examination value) in the case of therapeutic drug B and dose: 150 mg is higher than that in the case of therapeutic drug A and dose: 100 mg in both the good group and the poor group. Thus, classifying the good group and the poor group according to the type and dose of therapeutic drug and displaying the graphs 140 to 143 for each type and dose of therapeutic drug is helpful in a case where the medical staff determines the type and dose of therapeutic drug.

Fig. 30 exemplifies a case in which the graph comparison display screen 21 is displayed by outputting the second distribution request on the elapsed date "0" that is a reference date. Therefore, the line graph 144 showing a time-series change in the amount of change of the examination value of the medical examination target patient from the reference value is discontinued after the elapsed date "0" since there is no examination value after the elapsed date "0". In the present invention, instead of displaying the graph comparison display screen 21 in a case where several days has passed from the reference date that is a treatment start date as described above, it is also possible to display the graph comparison display screen 21 on the reference date to determine a treatment policy after the reference date.

In each of the embodiments described above, groups into which comparative cases are to be classified are two groups of the good group and the poor group. However, the number of groups into which comparative cases are to be classified may be two or more. For example, comparative cases may be classified into three groups of a good group, a poor group, and an intermediate group, or may be classified into four groups of a best group, a second best group, a second poorest group, and a poorest group.

In a case where the number of groups into which comparative cases are to be classified are two or more, the determination unit 50 determines whether or not there is a significant difference between the two or more groups. In a case where it is determined that there is a significant difference in combinations of all groups, the screen display control unit 51 generates the graph comparison display screen 21. In a case where groups into which comparative cases are to be classified are three groups of a good group, a poor group, and an intermediate group, the intermediate group does not contribute much to the determination of the future medical condition of the medical examination target patient. Accordingly, for the intermediate group, no representative value may be calculated, and display for comparison with the examination value of the medical examination target patient may not be performed.

Examination values are not limited to the examination values of vital signs or the examination values of subject examinations exemplified in the embodiments described above. Examination values may include a measurement value indicating the feature of a lesion in an examination image, which is obtained by performing image analysis of an examination image, such as a computed tomography (CT) image, a magnetic resonance imaging (MRI) image, or a simple X-ray image. As examples of the measurement value, measurement values regarding pixel values such as the average, variance, maximum, and minimum values of pixel values in the region of a lesion, measurement values regarding the shape such as the position of the region of a lesion and the circularity of the outline of the region, and measurement values regarding the size such as the radius, area, and volume of the region of a lesion can be mentioned. In this case, on the medical examination data display screen 20, the thumbnail of the examination image may be displayed together with the graph showing the time-series change in the measurement value.

The hardware configuration of a computer, which forms the medical examination assistance server 11 corresponding to the medical examination assistance apparatus of the present invention, can be modified in various ways. For example, in order to improve the processing capacity or reliability, the medical examination assistance server 11 may be formed by a plurality of server computers that are separated from each other as hardware. Specifically, the functions of the reception unit 46 and the acquisition unit 47 and the functions of the classification unit 48, the calculation unit 49, the determination unit 50, and the screen display control unit 51 may be distributed in two server computers. In this case, the two server computers form a medical examination assistance apparatus. Alternatively, the medical record DB server 12 may be removed and the electronic medical record DB 14 may be provided in the medical examination assistance server 11 to integrate the medical examination assistance server 11 and the medical record DB server 12, and the CPU 27B of the medical examination assistance server 11 may have the function of the search unit 57.

In the first embodiment described above, the case has been exemplified in which the medical examination assistance server 11 generates the display screens 20 and 21 and the display screens 20 and 21 are reproduced on the client terminal 10 side based on the XML data of the display screens 20 and 21 from the medical examination assistance server 11 and are displayed on the display 29A. However, the representative value and the medical examination data of the medical examination target patient as a generation source of the display screens 20 and 21 may be transmitted from the medical examination assistance server 11 to the client terminal 10, and the display screens 20 and 21 may be generated on the client terminal 10 side. In this case, the screen display control unit 51 is constructed in the CPU 27A of the client terminal 10. In this case, the client terminal 10 and the medical examination assistance server 11 form a medical examination assistance apparatus.

Each of the functional units 46 to 51 may be constructed in the CPU 27A of the client terminal 10, and the client terminal 10 may be operated as a medical examination assistance apparatus. In this case, the reception unit 46 receives various distribution instructions, instead of various distribution requests, from the GUI control unit 35. In addition, the screen display control unit 51 outputs the generated display screens 20 and 21 to the GUI control unit 35.

Thus, the hardware configuration of a computer can be appropriately changed according to the required performance, such as processing capacity, safety, or reliability. Needless to say, in order to ensure the safety or reliability, an application program, such as the medical examination assistance program 45 and the DB program 55, may be duplicated or may be stored in a plurality of storage devices in a distributed manner, without being limited to hardware.

In each of the embodiments described above, the medical examination assistance system 2 constructed in one medical facility is exemplified, and the medical examination assistance server 11 is used in one medical facility. However, the medical examination assistance server 11 may be configured to be usable in a plurality of medical facilities.

In each of the embodiments described above, the medical examination assistance server 11 is communicably connected to the client terminal 10, which is installed in one medical facility, through the network 13, such as a LAN, and provides various functions corresponding to various requests from the client terminal 10. In order to make the medical examination assistance server 11 available in a plurality of medical facilities, the medical examination assistance server 11 is communicably connected to each of the client terminals 10 installed in the plurality of medical facilities, for example, through a wide area network (WAN), such as the Internet or a public communication network. Then, the medical examination assistance server 11 receives requests from the client terminals 10 in the plurality of medical facilities through the WAN, and provides various functions to each client terminal 10. In the case of using a WAN, it is preferable to construct a virtual private network (VPN) or to use a communication protocol with a high security level, such as hypertext transfer protocol secure (HTTPS), in consideration of information security.

In this case, the installation location and management entity of the medical examination assistance server 11 may be a data center managed by a company that is different from the medical facilities, or may be one of the plurality of medical facilities, for example.

In each of the embodiments described above, the determination result of the significant difference is used only in the case of determining whether or not to display the representative value so as to be comparable with the examination value of the medical examination target patient. However, the determination result of the significant difference itself may be provided to the medical staff. The form of providing the determination result of the significant difference to the medical staff is not limited to the distribution of each of the display screens 20 and 21 through the web exemplified in the first embodiment described above. For example, a DB storing a file in which a determination result of a significant difference is recorded may be provided, and a medical staff may have a right to access the DB to read the file from the DB. The file may be automatically transmitted to the client terminal 10 using a known file transfer protocol, such as a file transfer protocol over SSL/TLS (FTPS). E-mail may be used instead of the file transfer protocol. In addition, it is also possible to output a paper material on which the determination result of the significant difference is printed.

In the present invention, it is also possible to appropriately combine the above-described various embodiments or various modification examples. Without being limited to the embodiments described above, it is needless to say that various configurations can be adopted without departing from the scope of the present invention. In addition to the program, the invention also extends to a storage medium that stores the program.

**Claims**

1.  A medical examination assistance apparatus, comprising:

    an acquisition unit that acquires comparative cases, which are medical examination data of a target to be compared with a medical examination target patient, from a case database in which medical examination data including examination values registered in time series is registered for each patient;
    a classification unit that classifies the comparative cases into a plurality of groups according to a treatment outcome;
    a calculation unit that calculates a representative value, which represents each of the groups, based on the examination values of the comparative cases belonging to the same group;

a determination unit that determines whether or not there is a significant difference between the plurality of groups; and

a screen display control unit that performs control to display the representative value and the examination values of the medical examination target patient on a display screen so as to be comparable with each other in a case where the determination unit determines that there is a significant difference.

2. The medical examination assistance apparatus according to claim 1,

wherein the screen display control unit displays a graph showing a time-series change in the representative value and a graph showing a time-series change in the examination value of the medical examination target patient or a graph showing a time-series change in an amount of change of the representative value from a reference value and a graph showing a time-series change in an amount of change of the examination value of the medical examination target patient from a reference value, on the display screen, so as to be comparable with each other.

3. The medical examination assistance apparatus according to claim 2,

wherein a reference date for displaying the graphs so as to overlap each other along a time axis is set.

4. The medical examination assistance apparatus according to claim 3,

wherein the screen display control unit displays the graphs of a period before and after the reference date so as to overlap each other.

5. The medical examination assistance apparatus according to claim 1,

wherein the examination values have a plurality of items,
the calculation unit calculates the representative value for each of the plurality of items, and
the determination unit performs determination for each of the plurality of items.

6. The medical examination assistance apparatus according to claim 2,

wherein the medical examination data includes a clinical path that summarizes a treatment plan for each patient, and
the comparative cases are the medical examination data having the same clinical path as the medical examination target patient.

7. The medical examination assistance apparatus according to claim 6,

wherein an application start date of the clinical path is set as a reference date for displaying the graphs so as to overlap each other along a time axis.

8. The medical examination assistance apparatus according to claim 2,

wherein the medical examination data includes contents of surgery performed on a patient, and
the comparative cases are the medical examination data having the same surgical contents as the medical examination target patient.

9. The medical examination assistance apparatus according to claim 8,

wherein a date of the surgery is set as a reference date for displaying the graphs so as to overlap each other along a time axis.

10. The medical examination assistance apparatus according to claim 2,

wherein the medical examination data includes a disease name of a patient and a therapeutic drug administered to a patient, and
the comparative cases are the medical examination data having the same disease name and therapeutic drug as the medical examination target patient.

11. The medical examination assistance apparatus according to claim 10,

wherein an administration start date of the therapeutic drug is set as a reference date for displaying the graphs so as to overlap each other along a time axis.

12. The medical examination assistance apparatus according to claim 1,

wherein the comparative cases are determined according to a degree of similarity with the examination value of the medical examination target patient.

13. The medical examination assistance apparatus according to claim 1,

wherein the classification unit classifies the comparative cases according to a length of a treatment period.

14. The medical examination assistance apparatus according to claim 1,

wherein the medical examination data includes a clinical path that summarizes a treatment plan for each patient and a variance that does not conform to the treatment plan, and
the classification unit classifies the comparative cases according to the variance.

15. The medical examination assistance apparatus according to claim 1,

wherein the determination unit performs determination based on a statistic of the examination values of the comparative cases of each of the plurality of groups.

16. The medical examination assistance apparatus according to claim 15,

wherein the statistic is the number of examination values of the comparative cases and an average value and a variance of the examination values of the comparative cases, and
the determination unit performs determination by t test for testing whether or not there is a significant difference in the average value of each of the plurality of groups.

17. The medical examination assistance apparatus according to claim 1,

wherein the screen display control unit does not display the representative value while the examination value of the medical examination target patient is within a specified range defined by the examination values of the comparative cases, which are classified into the group with the best treatment outcome by the classification unit, and displays the representative value in a case where the examination value of the medical examination target patient is outside the specified range.

18. The medical examination assistance apparatus according to claim 1,

wherein the screen display control unit displays a normal range of the examination value on the display screen.

19. An operation method of a medical examination assistance apparatus, comprising:

an acquisition step of acquiring comparative cases, which are medical examination data of a target to be compared with a medical examination target patient, from a case database in which medical examination data including examination values registered in time series is registered for each patient;
a classification step of classifying the comparative cases into a plurality of groups according to a treatment outcome;
a calculation step of calculating a representative value, which represents each of the groups, based on the examination values of the comparative cases belonging to the same group;
a determination step of determining whether or not there is a significant difference between the plurality of groups; and
a screen display control step of performing control to display the representative value and the examination values of the medical examination target patient on a display screen so as to be comparable with each other in a case where there is a significant difference in the determination step.

20. A medical examination assistance system, comprising:

the medical examination assistance apparatus according to any one of claims 1 to 18; and
a client terminal connected to the medical examination assistance apparatus through a network.

# FIG. 1

# FIG. 2

FIG. 2 — Block diagram showing the following elements and message flows:

**10 — CLIENT TERMINAL**

**11 — MEDICAL EXAMINATION ASSISTANCE SERVER**

**12 — MEDICAL RECORD DB SERVER**

From CLIENT TERMINAL to MEDICAL EXAMINATION ASSISTANCE SERVER:
FIRST DISTRIBUTION REQUEST PATIENT ID: P001

From MEDICAL EXAMINATION ASSISTANCE SERVER to MEDICAL RECORD DB SERVER:
FIRST ACQUISITION REQUEST PATIENT ID: P001

From MEDICAL RECORD DB SERVER to MEDICAL EXAMINATION ASSISTANCE SERVER:
MEDICAL EXAMINATION DATA OF MEDICAL EXAMINATION TARGET PATIENT

From MEDICAL EXAMINATION ASSISTANCE SERVER to CLIENT TERMINAL:
MEDICAL EXAMINATION DATA DISPLAY SCREEN — 20

From CLIENT TERMINAL to MEDICAL EXAMINATION ASSISTANCE SERVER:
SECOND DISTRIBUTION REQUEST EXAMINATION VALUE: ALB

From MEDICAL EXAMINATION ASSISTANCE SERVER to MEDICAL RECORD DB SERVER:
SECOND ACQUISITION REQUEST CPID: 001

From MEDICAL RECORD DB SERVER to MEDICAL EXAMINATION ASSISTANCE SERVER:
COMPARATIVE CASE

From MEDICAL EXAMINATION ASSISTANCE SERVER to CLIENT TERMINAL:
GRAPH COMPARISON DISPLAY SCREEN — 21

EP 3 276 571 A1

# FIG. 3

14

| PATIENT ID | ELECTRONIC |
|---|---|
| P001 | MEDICAL RECORD |

| PATIENT ID | ELECTRONIC |
|---|---|
| P002 | MEDICAL RECORD |

| PATIENT ID | ELECTRONIC |
|---|---|
| P003 | MEDICAL RECORD |

| DATE | BLOOD PRESSURE (MAXIMUM) |
|---|---|
| 02.02.2015 | 196 |
| 02.03.2015 | 180 |
| 02.04.2015 | 175 |

| DATE | BLOOD PRESSURE (MINIMUM) |
|---|---|
| 07/22/2014 | 92 |
| 07/23/2014 | 84 |
| 07/24/2014 | 79 |

| DATE AND TIME | BODY TEMPERATURE |
|---|---|
| 02.02.2015 | 38.9 |
| 02.03.2015 | 37.6 |
| 02.04.2015 | 36.9 |

| DATE | BIOCHEMICAL EXAMINATION |
|---|---|
| 02.02.2015 | AST/49 ALP/452 CREATININE/1.4··· |
| 02.03.2015 | AST/41 ALP/395 CREATININE/1.1··· |
| 02.04.2015 | AST/36 ALP/323 CREATININE/0.8··· |

| DATE | BLOOD TEST |
|---|---|
| 02.02.2015 | WBC/2.63 RBC/9.25 Ht/58.8 ALB/4.9··· |
| 02.03.2015 | WBC/3.77 RBC/7.63 Ht/52.4 ALB/4.9··· |
| 02.04.2015 | WBC/4.12 RBC/5.67 Ht/51.1 ALB/4.7··· |

| DATE | THERAPEUTIC DRUG A |
|---|---|
| 02.02.2015 | 100 |
| 02.03.2015 | 100 |
| 02.04.2015 | 50 |

| DATE | MEDICAL EXAMINATION AND TREATMENT RECORD |
|---|---|
| 02.02.2015 | CHIEF COMPLAINT: ABDOMINAL PAIN, NAUSEA<br>ORDER OF MEDICAL EXAMINATIONS: BIOCHEMISTRY, BLOOD, SIMPLE X-RAY IMAGING, AND ENDOSCOPIC EXAMINATION<br>DIAGNOSED DISEASE NAME: ACUTE GASTRIC ULCER<br>ADMISSION TO HOSPITAL ADMINISTRATION: THERAPEUTIC DRUG A |
| 02.03.2015 | ORDER OF MEDICAL EXAMINATIONS: BIOCHEMISTRY, BLOOD, SIMPLE X-RAY IMAGING<br>ADMINISTRATION: THERAPEUTIC DRUG A |
| 02.04.2015 | SURGERY ORDER, EXPLANATION<br>ORDER OF MEDICAL EXAMINATIONS: BIOCHEMISTRY, BLOOD<br>ABROSIA: MORNING, NOON SURGERY: ENDOSCOPIC MUCOSAL RESECTION |

# FIG. 4

10, 11, 12

DISPLAY — 29

INPUT DISPLAY — 30

27

CPU

31

MEMORY — 26

25

STORAGE DEVICE

COMMUNICATION UNIT — 28

13

NETWORK

# FIG. 5

29A

DISPLAY

30A

INPUT DISPLAY

GUI CONTROL UNIT — 35

BROWSER CONTROL UNIT — 36

DISPLAY SCREEN

20, 21

27A

FIRST AND SECOND DISTRIBUTION REQUESTS

FIG. 6

FIG. 7

FIRST AND SECOND
DISTRIBUTION REQUESTS

55 — DB PROGRAM — 25C

56

RECEPTION
UNIT

57

SEARCH UNIT

58

OUTPUT
CONTROL UNIT

27C

14

MEDICAL
EXAMINATION DATA OF
MEDICAL EXAMINATION
TARGET PATIENT

COMPARATIVE CASE

# FIG. 8

ACQUISITION UNIT    47

SECOND DISTRIBUTION REQUEST
CPID: CP050

COMPARATIVE CASES

| PATIENT ID | ELECTRONIC MEDICAL RECORD (CPID: CP050) |
|---|---|
| P005 | |

| PATIENT ID | ELECTRONIC MEDICAL RECORD (CPID: CP050) |
|---|---|
| P008 | |

| PATIENT ID | ELECTRONIC MEDICAL RECORD (CPID: CP050) |
|---|---|
| P020 | |

| PATIENT ID | ELECTRONIC MEDICAL RECORD (CPID: CP050) |
|---|---|
| P025 | |

14

12

FIG. 9

EP 3 276 571 A1

# FIG. 10

**COMPARATIVE CASES**

| PATIENT ID | ELECTRONIC MEDICAL RECORD |
|---|---|
| P005 | ELECTRONIC MEDICAL RECORD (CP APPLICATION START DATE: 05.15.2010, DISCHARGE DATE: 05.22.2010) |
| P008 | ELECTRONIC MEDICAL RECORD (CP APPLICATION START DATE: 07.22.2010, DISCHARGE DATE: 08.10.2010) |
| P020 | ELECTRONIC MEDICAL RECORD (CP APPLICATION START DATE: 09.14.2012, DISCHARGE DATE: 09.27.2010) |
| P025 | ELECTRONIC MEDICAL RECORD (CP APPLICATION START DATE: 04.12.2012, DISCHARGE DATE: -, DATE OF BIRTH: 04.16.2012) |

⋮

**48**

CLASSIFICATION UNIT
TREATMENT PERIOD ⇔
THRESHOLD VALUE (15 DAYS)

**65**

| PATIENT ID | TREATMENT PERIOD |
|---|---|
| P005 | 8 DAYS |
| P008 | 20 DAYS |
| P020 | 14 DAYS |
| P025 | - (DEAD) |

**GOOD GROUP**
(TREATMENT PERIOD LESS THAN 15 DAYS)

| PATIENT ID | ELECTRONIC MEDICAL RECORD |
|---|---|
| P005 | ELECTRONIC MEDICAL RECORD |
| P020 | ELECTRONIC MEDICAL RECORD |

⋮

**POOR GROUP**
(TREATMENT PERIOD 15 DAYS OR MORE OR DEAD)

| PATIENT ID | ELECTRONIC MEDICAL RECORD |
|---|---|
| P008 | ELECTRONIC MEDICAL RECORD |
| P025 | ELECTRONIC MEDICAL RECORD |

⋮

TO CALCULATION UNIT

EP 3 276 571 A1

# FIG. 11

## FIG. 12

69

⟨ELAPSED DATE: -1⟩

| | GOOD GROUP | POOR GROUP |
|---|---|---|
| NUMBER OF CASES | 7 | 10 |
| AVERAGE VALUE (REPRESENTATIVE VALUE) | 5.5 | 5.0 |
| VARIANCE | 0.052 | 0.064 |

⟨ELAPSED DATE: 0⟩

| | GOOD GROUP | POOR GROUP |
|---|---|---|
| NUMBER OF CASES | 7 | 10 |
| AVERAGE VALUE (REPRESENTATIVE VALUE) | 4.4 | 4.9 |
| VARIANCE | 1.469 | 3.602 |

⋮

# FIG. 13

**69**

〈ELAPSED DATE: -1〉

| | GOOD GROUP | POOR GROUP |
|---|---|---|
| NUMBER OF CASES | 7 | 10 |
| AVERAGE VALUE (REPRESENTATIVE VALUE) | 5.5 | 5.0 |
| VARIANCE | 0.052 | 0.064 |

〈ELAPSED DATE: 0〉

| | GOOD GROUP | POOR GROUP |
|---|---|---|
| NUMBER OF CASES | 7 | 10 |
| AVERAGE VALUE (REPRESENTATIVE VALUE) | 4.4 | 4.9 |
| VARIANCE | 1.469 | 3.602 |

⋮

**50**

DETERMINATION UNIT

**70**

〈ELAPSED DATE: -1〉

t VALUE = 17.14

〈ELAPSED DATE: 0〉

t VALUE = -0.369

⋮

**71**

〈ELAPSED DATE: -1〉

| t VALUE | = 17.14 > THRESHOLD VALUE = 2.131

⇓

THERE IS SIGNIFICANT DIFFERENCE

〈ELAPSED DATE: 0〉

| t VALUE | = 0.369 ≦ THRESHOLD VALUE = 2.131

⇓

THERE IS NO SIGNIFICANT DIFFERENCE

⋮

TO SCREEN DISPLAY CONTROL UNIT

# FIG. 14

FIG. 15

# FIG. 16

GRAPH COMPARISON DISPLAY SCREEN

EXAMINATION VALUE: ALB — 97A

MEDICAL EXAMINATION TARGET PATIENT
GOOD CASE AVERAGE — 95
POOR CASE AVERAGE

ELAPSED DATE

EXAMINATION VALUE: BODY TEMPERATURE — 97B

MEDICAL EXAMINATION TARGET PATIENT
GOOD CASE AVERAGE — 95
POOR CASE AVERAGE

ELAPSED DATE

EXAMINATION VALUES WBC AND RBC ARE NOT GRAPH-DISPLAYED SINCE THERE IS NO SIGNIFICANT DIFFERENCE

## FIG. 17

〈CLIENT TERMINAL〉

START

S100 — ISSUE SECOND DISTRIBUTION REQUEST

S110 — RECEIVE GRAPH COMPARISON DISPLAY SCREEN

S120 — DISPLAY GRAPH COMPARISON DISPLAY SCREEN

END

〈MEDICAL EXAMINATION ASSISTANCE SERVER〉

START

S200 — RECEIVE SECOND DISTRIBUTION REQUEST

S210 — ISSUE SECOND ACQUISITION REQUEST

S220 — ACQUIRE MEDICAL EXAMINATION DATA OF MEDICAL EXAMINATION TARGET PATIENT AND COMPARATIVE CASES (ACQUISITION STEP)

S230 — CLASSIFY COMPARATIVE CASES INTO GOOD GROUP AND POOR GROUP (CLASSIFICATION STEP)

S240 — CALCULATE REPRESENTATIVE VALUE (CALCULATION STEP)

S250 — DETERMINE WHETHER OR NOT THERE IS SIGNIFICANT DIFFERENCE BETWEEN RESPECTIVE GROUPS (DETERMINATION STEP)

S260 — OUTPUT GRAPH COMPARISON DISPLAY SCREEN (SCREEN DISPLAY CONTROL STEP)

END

〈MEDICAL RECORD DB SERVER〉

START

S300 — RECEIVE SECOND ACQUISITION REQUEST

S310 — SEARCH FOR MEDICAL EXAMINATION DATA OF MEDICAL EXAMINATION TARGET PATIENT AND COMPARATIVE CASES

S320 — OUTPUT MEDICAL EXAMINATION DATA OF MEDICAL EXAMINATION TARGET PATIENT AND COMPARATIVE CASES

END

## FIG. 18

REFERENCE DATE: SURGERY DATE

**48** CLASSIFICATION UNIT

**49** CALCULATION UNIT

**50** DETERMINATION UNIT

**51** SCREEN DISPLAY CONTROL UNIT

**21**

**47** ACQUISITION UNIT

SECOND ACQUISITION REQUEST SURGERY: TOTAL GASTRECTOMY

### COMPARATIVE CASES

| PATIENT ID | ELECTRONIC MEDICAL RECORD (SURGERY: TOTAL GASTRECTOMY) |
|---|---|
| P007 | |

| PATIENT ID | ELECTRONIC MEDICAL RECORD (SURGERY: TOTAL GASTRECTOMY) |
|---|---|
| P012 | |

| PATIENT ID | ELECTRONIC MEDICAL RECORD (SURGERY: TOTAL GASTRECTOMY) |
|---|---|
| P024 | |

| PATIENT ID | ELECTRONIC MEDICAL RECORD (SURGERY: TOTAL GASTRECTOMY) |
|---|---|
| P028 | |

**12** **14**

## FIG. 19

# FIG. 20

100

| DISEASE TYPE | DISEASE NAME | THERAPEUTIC DRUG |
|---|---|---|
| STANDARD PNEUMONIA | PNEUMOCOCCAL PNEUMONIA | PENICILLIN TYPE A, B, C, ···, CEPHEM TYPE A, B, C, ··· |
| | KLEBSIELLA PNEUMONIA PNEUMONIA | PENICILLIN TYPE A, B, C, ···, CEPHEM TYPE A, B, C, ··· |
| | STAPHYLOCOCCUS AUREUS PNEUMONIA | METHICILLON X, Y, Z, VANCOMYCIN X, Y, Z |
| ATYPICAL PNEUMONIA | PSEUDOMONAS AERUGINOSA PNEUMONIA | STREPTOMYCIN L, M, N, ··· |
| | MYCOPLASMA PNEUMONIA | MACROLIDE TYPE P, Q, R, ···, TETRACYCLINE TYPE P, Q, R, ··· |
| | CHLAMYDIA PNEUMONIA | MACROLIDE TYPE P, Q, R, ···, TETRACYCLINE TYPE P, Q, R, ··· |

# FIG. 21

# FIG. 22

# FIG. 23

<MEDICAL EXAMINATION DATA OF MEDICAL EXAMINATION TARGET PATIENT>

| PATIENT ID | ELECTRONIC MEDICAL RECORD |
|---|---|
| P200 | |

| DATE | BLOOD TEST |
|---|---|
| 02.02.2015 | ‥ALBUMIN/5. 8‥ |
| 02.03.2015 | ‥ALBUMIN/5. 5‥ |
| 02.04.2015 | ‥ALBUMIN/5. 2‥ |
| 02.05.2015 | ‥ALBUMIN/4. 3‥ |

XI

<MEDICAL EXAMINATION DATA OF MEDICAL EXAMINATION TARGET PATIENT>

| PATIENT ID | ELECTRONIC MEDICAL RECORD |
|---|---|
| P035 | |

| DATE | BLOOD TEST |
|---|---|
| 09.13.2015 | ‥ALBUMIN/5. 5‥ |
| 09.14.2015 | ‥ALBUMIN/5. 1‥ |
| 09.15.2015 | ‥ALBUMIN/4. 2‥ |
| 09.16.2015 | ‥ALBUMIN/4. 8‥ |

YI

57

SEARCH UNIT

$$\text{DEGREE OF SIMILARITY} = \Sigma WI \mid XI - YI \mid$$

COMPARE DEGREE OF SIMILARITY WITH THRESHOLD VALUE, DETERMINE SIMILARITY

⇓

DETERMINE SIMILAR MEDICAL EXAMINATION DATA AS COMPARATIVE CASES

FIG. 24

COMPARATIVE CASES

| PATIENT ID | ELECTRONIC MEDICAL RECORD |
|---|---|
| P006 | (CPID: CP100, VARIANCE: EXTENSION OF DIETARY RESTRICTION) |

| PATIENT ID | ELECTRONIC MEDICAL RECORD |
|---|---|
| P018 | (CPID: CP100, VARIANCE: NONE) |

| PATIENT ID | ELECTRONIC MEDICAL RECORD |
|---|---|
| P021 | (CPID: CP100, VARIANCE: ADDITION OF BLOOD TEST) |

| PATIENT ID | ELECTRONIC MEDICAL RECORD |
|---|---|
| P036 | (CPID: CP100, VARIANCE: REVALUATION OF ADMINISTRATION PERIOD) |

48

CLASSIFICATION UNIT

GOOD GROUP
(NO VARIANCE, OR POSITIVE VARIANCE)

| PATIENT ID | ELECTRONIC MEDICAL RECORD |
|---|---|
| P018 | |

| PATIENT ID | ELECTRONIC MEDICAL RECORD |
|---|---|
| P036 | |

POOR GROUP
(NEGATIVE VARIANCE, OR DEAD)

| PATIENT ID | ELECTRONIC MEDICAL RECORD |
|---|---|
| P006 | |

| PATIENT ID | ELECTRONIC MEDICAL RECORD |
|---|---|
| P021 | |

CALCULATION UNIT

# FIG. 25

| ELAPSED DATE | P005 | P020 | | AVERAGE VALUE (REPRESENTATIVE VALUE) | STANDARD DEVIATION |
|---|---|---|---|---|---|
| -1 | 5.2 | 5.5 | | 5.5 | 0.227 |
| 0 | 4.2 | 4.3 | | 4.4 | 1.212 |
| 1 | 3.8 | 4.2 | | 4.7 | 0.852 |
| 2 | 4.5 | 4.8 | | 4.1 | 1.003 |

67A

110

49
CALCULATION UNIT

| DATE | GRAPH COMPARISON DISPLAY SCREEN |
|---|---|
| 03.10.2015 | NOT GENERATED (NOT DISPLAYED) |
| 03.11.2015 | NOT GENERATED (NOT DISPLAYED) |
| 03.12.2015 | NOT GENERATED (NOT DISPLAYED) |
| 03.13.2015 | GENERATED (DISPLAYED) |

51

SCREEN DISPLAY CONTROL UNIT
EXAMINATION VALUE OF MEDICAL EXAMINATION TARGET PATIENT ⇔ SPECIFIED RANGE

| ELAPSED DATE | SPECIFIED RANGE (AVERAGE VALUE ±STANDARD DEVIATION) |
|---|---|
| -1 | 5.273 TO 5.727 |
| 0 | 3.188 TO 5.612 |
| 1 | 3.848 TO 5.552 |
| 2 | 3.097 TO 5.103 |

111

<MEDICAL EXAMINATION DATA OF MEDICAL EXAMINATION TARGET PATIENT>

| PATIENT ID | ELECTRONIC MEDICAL RECORD |
|---|---|
| P040 | |

| DATE | BLOOD TEST |
|---|---|
| 03.10.2015 | ··ALB/5.7·· |
| 03.11.2015 | ··ALB/5.4·· |
| 03.12.2015 | ··ALB/4.2·· |
| 03.13.2015 | ··ALB/3.0·· |

## FIG. 26

<MEDICAL EXAMINATION DATA OF MEDICAL
EXAMINATION TARGET PATIENT>

| PATIENT ID | ELECTRONIC |
|---|---|
| P040 | MEDICAL RECORD |

| DATE | BLOOD TEST |
|---|---|
| 03.10.2015 | ··ALB/5.7·· |
| 03.11.2015 | ··ALB/5.4·· |
| 03.12.2015 | ··ALB/4.2·· |
| 03.13.2015 | ··ALB/3.0·· |

CALCULATION UNIT 49

| ELAPSED DATE | EXAMINATION VALUE: ALB |
|---|---|
| -1 | 0 (REFERENCE VALUE) |
| 0 | -0.3 |
| 1 | -1.5 |
| 2 | -2.7 |

120

TO SCREEN DISPLAY CONTROL UNIT

EP 3 276 571 A1

# FIG. 27

**GOOD GROUP**

| PATIENT ID | ELECTRONIC MEDICAL RECORD |
|---|---|
| P005 | |

| DATE | BLOOD TEST |
|---|---|
| 05.14.2010 | ··ALB/5.2·· |
| 05.15.2010 | ··ALB/4.2·· |
| 05.16.2010 | ··ALB/3.8·· |
| 05.17.2010 | ··ALB/4.5·· |

| PATIENT ID | ELECTRONIC MEDICAL RECORD |
|---|---|
| P005 | |

| DATE | BLOOD TEST |
|---|---|
| 09.13.2012 | ··ALB/5.5·· |
| 09.14.2012 | ··ALB/4.3·· |
| 09.15.2012 | ··ALB/4.2·· |
| 09.16.2012 | ··ALB/4.8·· |

● ● ●

**49**
CLASSIFICATION UNIT

**67A**

| ELAPSED DATE | P005 | P020 | |
|---|---|---|---|
| -1 | 5.2 | 5.5 | |
| 0 | 4.2 | 4.3 | |
| 1 | 3.8 | 4.2 | |
| 2 | 4.5 | 4.8 | |

**121**

| ELAPSED DATE | P005 | P020 | |
|---|---|---|---|
| -1 | 0 (REFERENCE VALUE) | | |
| 0 | -1.0 | -1.2 | |
| 1 | -1.4 | -1.3 | |
| 2 | -0.7 | -0.7 | |

**122**

| AVERAGE VALUE (REPRESENTATIVE VALUE) |
|---|
| 0 |
| -1.1 |
| -1.5 |
| -0.8 |

TO SCREEN DISPLAY CONTROL UNIT

EP 3 276 571 A1

# FIG. 28

GRAPH COMPARISON DISPLAY SCREEN

AMOUNT OF CHANGE   91

EXAMINATION VALUE: ALB  —97

80

1.5

1.0

0.5

0

-0.5

-1.0

-1.5

-1   0                5                10               15

MEDICAL EXAMINATION TARGET PATIENT
GOOD CASE AVERAGE  —95
POOR CASE AVERAGE

130

131

132

ELAPSED DATE

# FIG. 29

COMPARATIVE CASES

CLASSIFICATION UNIT 48

THERAPEUTIC DRUG A, DOSE: 100 mg

GOOD GROUP    POOR GROUP

THERAPEUTIC DRUG B, DOSE: 150 mg

GOOD GROUP    POOR GROUP

## FIG. 30

## INTERNATIONAL SEARCH REPORT

| | International application No. |
|---|---|
| | PCT/JP2016/056949 |

**A. CLASSIFICATION OF SUBJECT MATTER**
*G06Q50/22(2012.01)i*

According to International Patent Classification (IPC) or to both national classification and IPC

**B. FIELDS SEARCHED**

Minimum documentation searched (classification system followed by classification symbols)
G06Q50/22

Documentation searched other than minimum documentation to the extent that such documents are included in the fields searched
Jitsuyo Shinan Koho        1922-1996    Jitsuyo Shinan Toroku Koho    1996-2016
Kokai Jitsuyo Shinan Koho  1971-2016    Toroku Jitsuyo Shinan Koho    1994-2016

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2014-109836 A  (Fujifilm Corp.), 12 June 2014 (12.06.2014), paragraphs [0010], [0014], [0020], [0058] to [0061], [0069]; fig. 4, 9 & US 2015/0254430 A1 paragraphs [0012], [0016], [0022], [0079] to [0082], [0090]; fig. 4, 9 & WO 2014/084294 A1 paragraphs [0009], [0014], [0019], [0058] to [0061], [0069]; fig. 4, 9 & CN 104823211 A | 1-20 |
| Y | JP 2015-18462 A  (Hitachi, Ltd.), 29 January 2015 (29.01.2015), claim 1; paragraphs [0012], [0045] (Family: none) | 1-20 |

☒ Further documents are listed in the continuation of Box C.      ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 11 April 2016 (11.04.16) | 19 April 2016 (19.04.16) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

International application No.

PCT/JP2016/056949

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2014-35701 A  (Canon Inc.),<br>24 February 2014 (24.02.2014),<br>paragraphs [0029] to [0030]<br>(Family: none) | 6-9 |
| Y | JP 2013-198817 A  (Canon Inc.),<br>03 October 2013 (03.10.2013),<br>abstract; paragraphs [0145] to [0146]<br>(Family: none) | 10-12 |
| Y | JP 2011-105677 A  (Terumo Corp.),<br>02 June 2011 (02.06.2011),<br>paragraph [0034]<br>(Family: none) | 13 |
| Y | JP 2005-165513 A  (Olympus Corp.),<br>23 June 2005 (23.06.2005),<br>paragraph [0070]<br>& US 2005/0159981 A1<br>paragraph [0306] | 14 |
| Y | WO 2009/090882 A1  (THE UNIVERSITY OF TOKYO),<br>23 July 2009 (23.07.2009),<br>paragraph [0089]<br>(Family: none) | 15-16 |
| Y | JP 3-100773 A  (Shimadzu Corp.),<br>25 April 1991 (25.04.1991),<br>claims<br>(Family: none) | 17 |
| Y | JP 2015-35210 A  (Higashi Nihon Medicom Co.,<br>Ltd.),<br>19 February 2015 (19.02.2015),<br>paragraphs [0036] to [0037]; fig. 7, 8<br>(Family: none) | 18 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014109836 A